(19) Europäisches Patentamt
European Patent Office
Office européen des brevets

(11) Publication number: 0 093 589 A1

# (12) EUROPEAN PATENT APPLICATION

(21) Application number: 83302428.4

(22) Date of filing: 29.04.83

(51) Int. Cl.³: C 07 D 417/04
C 07 D 285/12, A 01 N 43/82

(30) Priority: 03.05.82 US 374138

(43) Date of publication of application: 09.11.83 Bulletin 83/45

(84) Designated Contracting States: BE CH DE FR GB IT LI LU NL SE

(71) Applicant: ELI LILLY AND COMPANY
307, East McCarty Street
Indianapolis Indiana 46285(US)

(72) Inventor: Thibault, Thomas Delor
1520 N. Franklin Road
Indianapolis Indiana 46219(US)

(74) Representative: Crowther, Terence Roger et al,
Erl Wood Manor
Windlesham Surrey GU20 6PH(GB)

(54) **Thiadiazolylimidazolidinones, intermediates, formulations and herbicidal method.**

(57) This invention discloses herbicidal thiadiazolylimidazolidinone derivatives of the formula

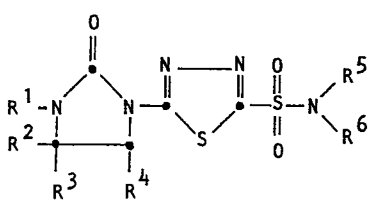

wherein $R^1$ is hydrogen, alkyl or phenyl, $R^2$ is hydrogen or a bond when taken together with $R^4$, $R^3$ is hydrogen or alkyl, $R^4$ is hydroxy, alkanoyloxy, alkoxy aminoalkanoyloxy or dialkylamino, $R^5$ is hydrogen or alkyl, and $R^6$ is hydrogen, alkyl, alkenyl, alkynyl or alkoxy, or $R^5$ and $R^6$ together complete a ring.

EP 0 093 589 A1

# THIADIAZOLYLIMIDAZOLIDINONES, INTERMEDIATES, FORMULATIONS AND HERBICIDAL METHOD

This invention relates to thiadiazolylimidazolidinone derivatives that are useful in the control and elimination of unwanted vegetation.

Certain thiadiazolylimidazolidinones are known in the art, and such compounds have been reported to be active as herbicidal agents. For example, Krenzer, in U.S. Patent No. 3,901,904, described certain thiadiazolylimidazolidinones having an alkyl group at the 1-position of the imidazolidinone group and a substituent at the 5 position of the thiadiazolyl group selected from alkyl, alkenyl, chloroalkyl, alkoxy, alkylthio, alkylsulfonyl and alkylsulfinyl.

The present invention provides thiadiazolylimidazolidinones wherein the thiadiazolyl group bears a sulfonamido group at the 5 position. These compounds exhibit desirable herbicidal activity.

More specifically, the present invention provides thiadiazolylimidazolidinone derivatives of the formula (I):

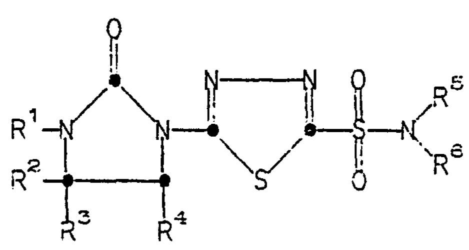

(I)

wherein:

$R^1$ is hydrogen, $C_1$-$C_6$ alkyl, or phenyl;

$R^2$ is hydrogen, or taken together with $R^4$ completes a double bond;

$R^3$ is hydrogen or $C_1$-$C_6$ alkyl;

$R^4$ is hydroxy, $C_1$-$C_6$ alkanoyloxy, $C_1$-$C_6$ alkoxy, di($C_1$-$C_6$ alkyl)amino, $O\overset{O}{\overset{\|}{C}}NR^1R^b$, where $R^b$ is hydrogen or $C_1$-$C_6$ alkyl and $R^1$ is as defined above; or taken together with $R^2$, $R^4$ completes a double bond;

$R^5$ is hydrogen or $C_1$-$C_6$ alkyl; and

$R^6$ is hydrogen, $C_1$-$C_6$ alkyl, $C_2$-$C_6$ alkenyl, $C_2$-$C_6$ alkynyl, $C_1$-$C_6$ alkoxy, or taken together with the nitrogen to which they are attached, $R^5$ and $R^6$ are pyrrolidino, piperidino or morpholino.

Preferred compounds have the above formula wherein $R^1$ is $C_1$-$C_6$ alkyl, $R^2$ and $R^3$ both are hydrogen, and $R^4$ is hydroxy or $C_1$-$C_6$ alkanoyloxy.

Also preferred are compounds wherein $R^5$ and $R^6$ independently are $C_1$-$C_6$ alkyl such as methyl or ethyl.

The most preferred compounds of the invention have the above formula wherein $R^1$ is methyl, $R^2$ and $R^3$ both are hydrogen, $R^4$ is hydroxy, and $R^5$ and $R^6$ independently are $C_1$-$C_6$ alkyl. Within this group, 3-(5-dimethylaminosulfonyl-1,3,4-thiadiazol-2-yl)-4-hydroxy-1-methyl-2-imidazolidinone is especially preferred.

Compounds of formula (I) wherein $R^2$ is hydrogen and $R^4$ is hydroxy or alkanoyloxy are referred to herein as "thiadiazolylimidazolidinones". The compounds of formula (I) wherein $R^2$ and $R^4$ together are a double bond are referred to as "thiadiazolylimidazolinones". The compounds provided by the invention are collectively referred to as "thiadiazolylimidazolidinone derivatives."

As used herein, the term "$C_1$-$C_6$ alkyl" carries its art-recognized meaning of straight and branched carbon chains having from one to six carbon atoms. Typical $C_1$-$C_6$ alkyl groups include methyl, ethyl, n-propyl, isopropyl, n-butyl, isobutyl, tert.-butyl, n-pentyl, n-hexyl, isohexyl, 1-ethyl-1-methylpropyl and the like.

Examples of "$C_1$-$C_6$ alkanoyloxy" include acetoxy, formyloxy, pentanoyloxy, 3-methylpentanoyloxy, butyroxy, and related groups.

The term "$C_1$-$C_6$ alkoxy" means a $C_1$-$C_6$ alkyl group linked through oxygen. Typical $C_1$-$C_6$ alkoxy groups include methoxy, ethoxy, isopropoxy, n-butoxy, n-hexyloxy, isohexyloxy and the like.

$R^4$ in formula (I), in addition to being hydroxy, alkanoyloxy or alkoxy, can be a "di($C_1$-$C_6$ alkyl)amino" group, which term refers to an amino group bearing two alkyl groups that are from 1 to 6 carbon atoms in length. Typical di($C_1$-$C_6$ alkyl)amino groups include dimethylamino, diethylamino and di-n-propylamino, as well as methylethylamino, methyl-n-butylamino and the like. $R^4$ in formula (I) also can be a urethane group represented by the formula $O\overset{O}{\overset{\|}{C}}NR^1R^b$, wherein $R^1$ is hydrogen $C_1$-$C_6$ alkyl or phenyl and $R^b$ is hydrogen or $C_1$-$C_6$ alkyl. Examples of such group include methylaminocarbonyloxy, dimethylaminocarbonyloxy, N-methylphenylaminocarbonyloxy, and hexylaminocarbonyloxy.

The compounds provided by this invention require a sulfonamido group attached at the 5 position

of the thiadiazolyl ring system. The sulfonamido group bears two substituents on the nitrogen atom defined as $R^5$ and $R^6$. $R^5$ can be hydrogen or $C_1$-$C_6$ alkyl and $R^6$ can be hydrogen, $C_1$-$C_6$ alkyl, $C_2$-$C_6$ alkenyl, $C_2$-$C_6$ alkynyl or $C_1$-$C_6$ alkoxy. The term "$C_2$-$C_6$ alkenyl" includes groups such as propenyl, 2-butenyl, 3-hexenyl and 1-methyl-2-pentenyl. The term "$C_2$-$C_6$ alkynyl" includes groups such as propynyl, 2-butynyl, 3-pentynyl, and 1-methyl-3-pentynyl. Typical $C_1$-$C_6$ alkoxy groups include methoxy, ethoxy, n-butoxy and isohexyloxy.

## Preparation of thiadiazolyl-4-hydroxy-imidazolidinones

The thiadiazolyl-4-hydroxy-imidazolidinones provided by this invention can be prepared by cyclization of a suitably substituted thiadiazolylurea of the formula

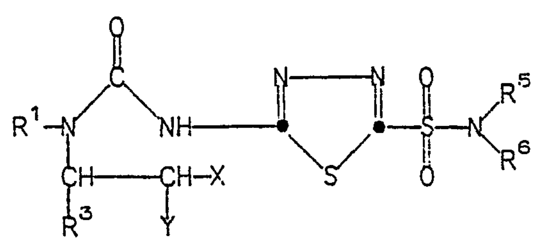

wherein $R^1$, $R^3$, $R^5$ and $R^6$ are as defined above, and X and Y are $C_1$-$C_6$ alkoxy such as methoxy, ethoxy, propoxy or the like.

The cyclization of the di-alkoxy thiadiazolyl urea intermediate can be accomplished by simply heating the intermediate in the presence of an acid. Commonly used acids include mineral acids such as hydrochloric, hydrobromic, sulfuric, phosphoric, nitric, and related acids. Organic acids such as acetic acid can also be

utilized if desired. The amount of acid commonly employed to effect the cyclization is about an equimolar amount or an excess, and routinely the reaction is simply carried out in a dilute aqueous acid solution having an acid concentration of about 0.5 to about 5 percent by weight. If desired, the cyclization reaction can be carried out in a solvent medium other than water, such as dioxane, dimethylsulfoxide, dimethylformamide, and the like, with an equimolar or excess amount of suitable acid added. The cyclization reaction generally is substantially complete within about 10 to about 90 minutes when carried out at a temperature of about 20 to about 100°C. Longer reaction times are not detrimental and can be employed if desired. The reaction provides a thiadiazolyl-4-hydroxyimidazolidinone (i.e. an imidazolidinone of the above formula wherein $R^4$ is hydroxy) which is readily isolated by cooling the reaction mixture, for instance to 0° to about 25°C, and then collecting the precipitate. The precipitated thiadiazolyl-4-hydroxyimidazolidinone can be further purified if desired by conventional means, including chromatography and crystallization from common solvents such as ethanol, acetone, dioxane, water and the like.

Examples of thiadiazolyl-4-hydroxyimidazolidinones which are readily prepared according to the above described cyclization process include the following:

3-(5-Dimethylaminosulfonyl-1,3,4-thiadiazol-2-yl)-4-hydroxy-1-ethyl-2-imidazolidinone;

3-(5-N-methyl-N-ethylamino-1,3,4-thiadiazolyl)-4-hydroxy-1-methyl-2-imidazolidinone;

3-(5-Diethylaminosulfonyl-1,3,4-thiadiazol-2-yl)-4-hydroxy-1-*n*-butyl-2-imidazolidinone;

3-(5-N-methyl-N-butylaminosulfonyl-1,3,4-thiadiazol-2-yl)-4-hydroxy-1-*n*-butyl-2-imidazolidinone;

3-(5-Di-n-butylaminosulfonyl-1,3,4-thiadiazol-2-yl)-4-hydroxy-1-methyl-2-imidazolidinone;

3-(5-Pyrrolidinosulfonyl-1,3,4-thiadiazol-2-yl)-4-hydroxy-1-ethyl-2-imidazolidinone;

3-(5-Morpholinosulfonyl-1,3,4-thiadiazol-2-yl)-4-hydroxy-1-methyl-2-imidazolidinone;

3-(5-Piperidinosulfonyl-1,3,4-thiadiazol-2-yl)-4-hydroxy-1-*n*-propyl-2-imidazolidinone;

3-[5-N-methyl-N-(2-propenyl)aminosulfonyl-1,3,4-thiadiazol-2]-4-hydroxy-1-methyl-2-imidazolidinone;

3-[5-N-ethyl-N-(2-propynyl)aminosulfonyl-1,3,4-thiadiazol-2-yl]-4-hydroxy-1-ethyl-2-imidazolidinone;

3-(5-N-methoxy-N-ethylaminosulfonyl-1,3,4-thiadiazol-2-yl)-4-hydroxy-1-ethyl-2-imidazolidinone; and

3-(5-N-hexyl-N-*iso*propoxyaminosulfonyl-1,3,4-thiadiazol-2-yl)-4-hydroxy-1-*n*-hexyl-2-imidazolidinone.

Thiadiazolylimidazolinone derivatives

The thiadiazolyl-4-hydroxyimidazolidinones thus described are useful not only as herbicides, but

also as intermediates in the synthesis of other imidazolidinone derivatives of the invention. For example, the unsaturated imidazolinones of the invention, i.e. compounds of the above formula wherein $R^2$ and $R^4$ together form a double bond, can be prepared by dehydrating a 4-hydroxyimidazolidinone according to the following sequence:

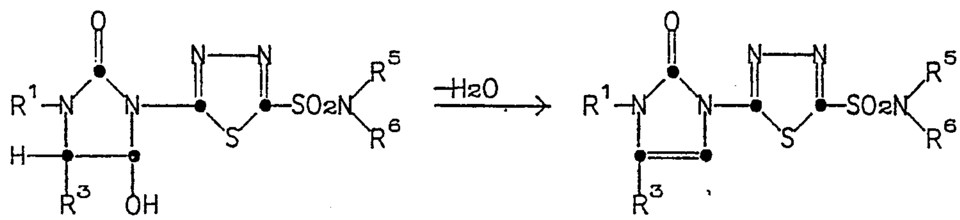


Such dehydration can be effected by reacting the 4-hydroxyimidazolidinone with an acid such as a mineral acid, or preferably with an equimolar amount or slight excess of a halogenating agent such as thionyl chloride. For example, reaction of a compound such as 3-(5-dimethylaminosulfonyl-1,3,4-thiadiazol-2-yl)-4-hydroxy-1-ethyl-2-imidazolidinone with about an equimolar amount of thionyl chloride in a solvent such as chloroform or methylene chloride for about 1 to about 24 hours at a temperature of about 0 to about 50°C provides the corresponding unsaturated imidazolinone, namely 3-(5-dimethylaminosulfonyl-1,3,4-thiadiazol-2-yl)-1-ethyl-2-imidazolinone. The product can be recovered by simply removing the reaction solvent, for instance by evaporation, and further purification can be achieved if desired by routine procedures such as crystallization from common solvents such acetone or diethyl ether.

## 4-Alkanoyloxy derivatives

The thiadiazolyl-4-hydroxyimidazolidinones provided by this invention are also useful in the preparation of those imidazolidinones of general formula (I) wherein $R^4$ is $C_1$-$C_6$ alkanoyloxy. For example, the thiadiazolyl-4-hydroxyimidazolidinones are readily acylated with an acylating agent such as an acid halide. Commonly used acylating agents include acetyl chloride, butyryl iodide, hexanoyl bromide, and the like. Other acylating agents commonly employed include acid anhydrides such as acetic anhydride, acetic formic anhydride, propionic formic anhydride, and the like.

The acylation of a thiadiazolyl-4-hydroxyimidazolidinone can be accomplished by reacting about equimolar quantities of a 4-hydroxyimidazolidinone and an acylating agent. An excess of acylating agent can be employed if desired. The reaction typically is conducted in a solvent such as benzene, acetone, dichloromethane, chloroform, or the like, and routinely is carried out in the presence of a base to act as an acid scavenger. Commonly used bases include triethylamine, pyridine, sodium carbonate, and the like. The acylation reaction is generally substantially complete after about 2 to about 24 hours when carried out at a temperature of about -20 to about 10°C. The acylated product, namely a thiazolylimidazolidinone of the above formula wherein $R^4$ is $C_1$-$C_6$ alkanoyloxy, can be isolated by simply removing the reaction solvent, for instance by evaporation under reduced pressure. The acylated imidazolidinone can be purified if needed by conventional means, including washing with dilute acid and dilute base, chromatography, crystallization, and the like.

Urethane derivatives

The thiadiazolylimidazolidinone urethanes provided by this invention, compounds of formula (I) wherein $R^4$ is $\overset{O}{\overset{\|}{OC}}NR^1R^b$, are prepared by reaction of thiadiazolyl-4-hydroxyimidazolidinones with isocyanates of the formula $R^1NCO$ or a carbamoyl halide such as $R^1R^bN\overset{O}{\overset{\|}{C}}-Cl$. This acylation reaction generally is accomplished by combining the thiadiazolyl-4-hydroxyimidazolidinone with about an equal molar quantity or slight excess of an isocyanate or carbamoyl halide. The reaction generally is carried out in an organic solvent such as benzene, toluene, dichloromethane, chloroform, or the like, and normally is conducted at an elevated temperature of about 30 to about 80°C. At this temperature, the reaction generally is complete after about 2 to about 24 hours. The product generally is a solid that can be recovered by filtration. Further purification can be accomplished by routine methods such as crystallization or chromatography.

4-amino derivatives

The thiadiazolyl-4-hydroxyimidazolidinones provided by this invention also serve as intermediates in the preparation of imidazolidinone derivatives that have an amino group attached at the 4-position of the

imidazolidinone ring system. Such compounds are prepared by reaction of approximately equal molar quantities of an amine of the formula NHdi($C_1$-$C_6$ alkyl) and the appropriate 4-hydroxyimidazolidinone derivative. This reaction generally is carried out in an unreactive organic solvent such as tetrahydrofuran, diethyl ether, chloroform, or the like, and usually is complete within about 6 to about 24 hours when conducted at a temperature of about 40 to about 80°C. The product of this reaction can be isolated by removal of the reaction solvents, for instance by evaporation under reduced pressure followed by normal purification of the product by chromatography or crystallization.

4-Alkoxy derivatives

4-Alkoxyimidazolidinone derivatives provided by this invention, compounds having the above formula wherein $R^4$ is $C_1$-$C_6$ alkoxy, can be prepared by reaction of a 4-hydroxyimidazolidinone derivative with a $C_1$-$C_6$ alkyl alcohol in the presence of a mineral acid such as sulfuric acid. For example, reaction of a compound such as 3-(5-dimethylaminosulfonyl-1,3,4-thiadiazol-2-yl)-4-hydroxy-1-n-butyl-2-imidazolidinone with an alcohol such as ethanol in the presence of sulfuric acid at a temperature of about 60°C effects alkylation at the 4-hydroxy group to provide the corresponding 4-ethoxyimidazolidinone derivative. Isolation of the product is accomplished by removal of the reaction solvent.

Typical compounds comprehended by this invention which are conveniently prepared from 4-hydroxyimidazolidinones include the following:

3-(5-Dimethylaminosulfonyl-1,3,4-thiadiazol-2-yl)-4-acetoxy-1-n-propyl-2-imidazolidinone;

3-(5-N-ethyl-N-methylaminosulfonyl-1,3,4-thiadiazol-2-yl)-4-acetoxy-1-(1,1-dimethylpropyl)-2-imidazolidinone;

3-(5-Piperidinosulfonyl-1,3,4-thiadiazol-2-yl)-4-n-butyroxy-5-ethyl-1-methyl-2-imidazolidinone;

3-(5-Isopropylaminosulfonyl-1,3,4-thiadiazol-2-yl)-4-propionoxy-1-(1,1-dimethylbutyl)-2-imidazolidinone;

3-(5-Morpholinosulfonyl-1,3,4-thiadiazol-2-yl)-4-acetoxy-1-isopropyl-2-imidazolidinone;

3-[5-N-methyl-N-(2-butenyl)aminosulfonyl-1,3,4-thiadiazol-2-yl]-4-acetoxy-1-ethyl-2-imidazolidinone;

3-(5-N-methyl-N-methoxyaminosulfonyl-1,3,4-thiadiazol-2-yl)-4-acetoxy-1-methyl-2-imidazolidinone;

3-(5-Dimethylaminosulfonyl-1,3,4-thiadiazol-2-yl)-4-ethylaminocarbonyloxy-5-methyl-1-ethyl-2-imidazolidinone;

3-(5-Dimethylaminosulfonyl-1,3,4-thiadiazol-2-yl)-4-diethylaminocarbonyloxy-1-n-butyl-2-imidazolidinone;

3-(5-Isobutoxyaminosulfonyl-1,3,4-thiadiazol-2-yl)-4-phenylaminocarbonyloxy-1-ethyl-2-imidazolidinone;

3-(5-Pyrrolidinosulfonyl-1,3,4-thiadiazol-2-yl)-4-dimethylamino-1-tert.-butyl-2-imidazolidinone;

3-(5-Di-n-hexylaminosulfonyl-1,3,4-thiadiazol-2-yl)-4-N-methyl-N-ethylamino-5-ethyl-1-n-propyl-2-imidazolidinone;

3-(5-Dimethylaminosulfonyl-1,3,4-thiadiazol-2-yl)-4-acetoxy-1-(2-methylpentyl)-2-imidazolidinone;

3-(5-Morpholinosulfonyl-1,3,4-thiadiazol-2-yl)-4-*n*-butyroxy-5-ethyl-1-(1,1-dimethylpropyl)-2-imidazolidinone;

3-(5-Dimethylaminosulfonyl-1,3,4-thiadiazol-2-yl)-1-phenyl-2-imidazolinone;

3-[5-N-ethyl-N-(3-pentynyl)aminosulfonyl-1,3,4-thiadiazol-2-yl]-1-methyl-2-imidazolinone;

3-(5-Morpholinosulfonyl-1,3,4-thiadiazol-2-yl)-1-methyl-2-imidazolinone;

3-(5-N-methyl-N-hexylaminosulfonyl-1,3,4-thiadiazol-2-yl)-1-methyl-2-imidazolinone; and

3-(5-Dimethylaminosulfonyl-1,3,4-thiadiazol-2-yl)-5-methyl-1-(1,1-dimethylpropyl)-2-imidazolinone.

Accordingly, one aspect of the present invention is that it provides a process for preparing a thiadiazolylimidazolidinone derivative of formula (I) which comprises the steps of

(a) heating a (dialkoxy)ethyl thiadiazolylurea of the formula (II):

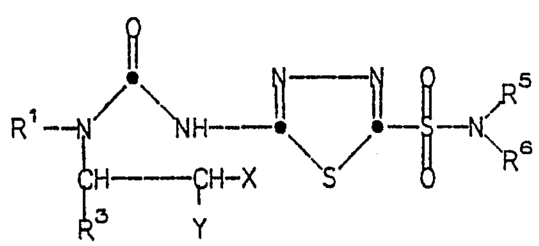

(II)

wherein:

$R^1$ is hydrogen, $C_1$-$C_6$ alkyl, or phenyl,

$R^3$ is hydrogen or $C_1$-$C_6$ alkyl,

$R^5$ is hydrogen or $C_1$-$C_6$ alkyl, and

$R^6$ is hydrogen, $C_1$-$C_6$ alkyl, $C_2$-$C_6$ alkenyl, $C_2$-$C_6$ alkynyl, $C_1$-$C_6$ alkoxy, or

$R^5$ and $R^6$ taken together with the nitrogen to which they are attacked form pyrrolidino, piperidino, or morpholino, and

X and Y independently are $C_1$-$C_6$ alkoxy; in the presence of an inorganic or an organic acid; or

(b) dehydrating a thiadiazolylimidazolidinone derivative of formula (I) wherein $R^4$ is hydroxy to provide a thiadiazolylimidazolidinone derivative of formula (I) wherein $R^2$ and $R^4$ combine to complete a double bond; or

(c) reacting a thiadiazolylimidazolidinone derivative of formula (I) wherein $R^4$ is hydroxy with an acylating agent derived from a $C_1$-$C_6$ carboxylic acid to provide a thiadiazolylimidazolidinone derivative of formula (I) wherein $R^4$ is $C_1$-$C_6$ alkanoyloxy; or

(d) reacting a thiadiazolylimidazolidinone derivative of formula (I) wherein $R^4$ is hydroxy with an isocyanate of the formula $R^1NCO$ or a carbamoyl halide of the formula $R^1R^6NCOCl$, wherein $R^1$ is as defined above and $R^6$ is hydrogen or $C_1$-$C_6$ alkyl, to provide a thiadiazolylimidazolidinone derivative of formula (I) wherein $R^4$ is $O\text{-}\overset{O}{\overset{\|}{C}}NR^1R^6$; or

(e) reacting a thiadiazolylimidazolidinone derivative of formula (I) wherein $R^4$ is hydroxy with an amine of the formula

$$di(C_1\text{-}C_6\ alkyl)\text{-}NH$$

to provide a thiadiazolylimidazolidinone derivative of formula (I) wherein $R^4$ is di($C_1$-$C_6$ alkyl)amino; or

(f) reacting a thiadiazolylimidazolidinone derivative of formula (I) wherein $R^4$ is hydroxy with a $C_1$-$C_6$ alkyl alcohol in the presence of a mineral acid to provide a thiadiazolylimidazolidinone derivative of formula (I) wherein $R^4$ is $C_1$-$C_6$ alkoxy.

Preparation of
thiadiazolylurea intermediate

As pointed out earlier, the thiadiazolylimidazolidinone derivatives of this invention require, as an initial starting material, a thiadiazolylurea of the formula (II)

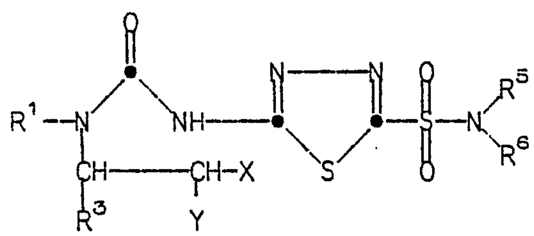

(II)

wherein X and Y are $C_1$-$C_6$ alkoxy groups such as methoxy, ethoxy or the like. The thiadiazolylurea intermediates can be prepared by any of several procedures. In a typical procedure, a 2-amino-5-aminosulfonyl-1,3,4-thiadiazole is reacted with a haloformate such as phenyl chloroformate to give a thiadiazolyl carbamate, which when reacted with a suitably substituted ethylamine gives the thiadiazolylurea of the invention. Such procedure can be illustrated by the following sequence:

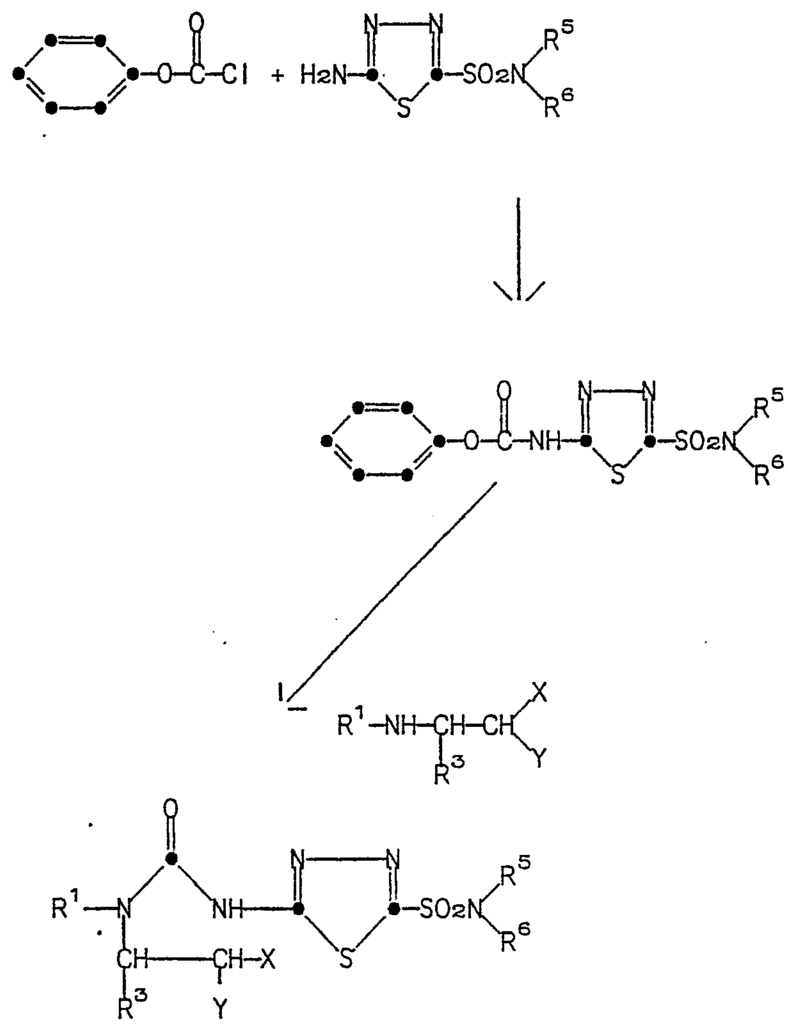

The 2-aminothiadiazole sulfonamides, wherein $R^5$ and $R^6$ are as defined above, are known in the art or are available by art known methods. Similarly, the dialkoxy ethylamines of the formula $R^1NHCH(R^3)CHXY$ are well known in the art and some are commercially available.

The reaction of a haloformate such as phenyl chloroformate and a 2-aminothiadiazole generally is

carried out by combining an excess of haloformate with the thiadiazole in an organic solvent such as pyridine or triethylamine, and stirring the mixture for about 1 to about 24 hours at a temperature of about 0 to about 30°C. The product of the reaction, a thiadiazolyl carbamate, is generally isolated by adding the reaction mixture to cold water and then acidifying the aqueous mixture, for instance by the addition of a mineral acid such as hydrochloric acid or sulfuric acid. The carbamate product generally precipitates out of solution and can be collected by filtration. The carbamate normally needs no further purification, but if desired it can be crystallized from common solvents such as benzene, acetone, ethyl acetate and the like.

The thiadiazolyl carbamate thus formed is next reacted with a 2,2-dialkoxy ethylamine derivative. Typical ethylamines commonly employed include N-methyl-2,2-dimethoxyethylamine, N-ethyl-2,2-diethoxyethylamine, N-isobutyl-1-methyl-2-ethoxy-2-methoxyethylamine, N-(phenyl)-1-n-hexyl-2-methoxy-2-hexyloxyethylamine, 2,2-diethoxyethylamine, and the like.

The thiadiazolyl carbamate and the ethylamine generally are combined in a mutual organic solvent such as toluene or benzene. The reactants can be utilized in about equimolar amounts, or if desired the ethylamine derivative can be employed in excess. The reaction routinely is substantially complete within about 2 to about 5 hours when carried out at a temperature of about 50 to about 100°C. Isolation of the product, a

thiadiazolylurea intermediate of the invention, usually is accomplished by filtration or by simply removing the reaction solvent, for instance by evaporation under reduced pressure. The thiadiazolylurea can be further purified if needed by routine methods, including recrystallization and chromatography.

An alternative procedure for preparing the thiadiazolylurea intermediates of the invention comprises reacting a suitably substituted 2,2-dialkoxy ethylamine derivative with a 2-(5-aminosulfonyl-1,3,4-thiadiazolyl isocyanate. The thiadiazolyl isocyanate is conveniently prepared by reacting a 2-aminothiadiazole with phosgene in the presence of an acid such as hydrochloric acid to give the corresponding 2-chlorocarbonylaminothiadiazole. The latter compound undergoes dehydrohalogenation in situ to provide the corresponding thiadiazolyl isocyanate. The reaction of the isocyanate with an ethylamine derivative is illustrated by the following sequence:

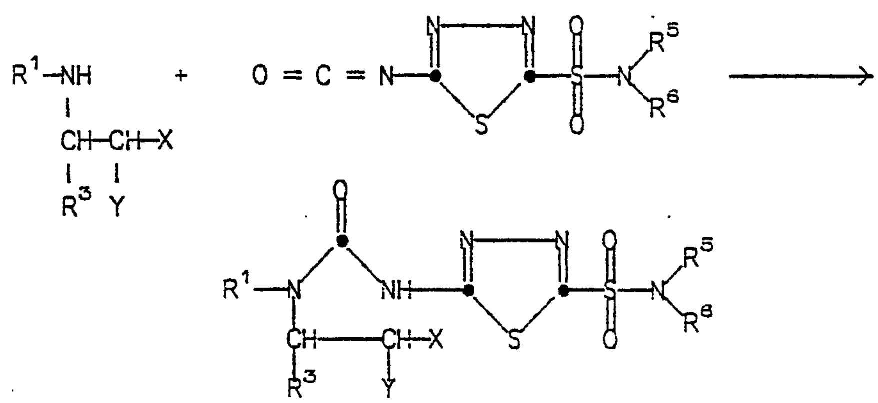

wherein $R^1$, $R^3$, $R^5$, $R^6$, X and Y are all as heretofore defined. The reaction of an ethylamine with a thiadiazolyl isocyanate generally is carried out by combining approximately equimolar quantities of the reactants in a suitable solvent such as benzene or toluene. The reaction normally is complete within about 2 to about 5 hours when carried out at a temperature of about 50 to about 100°C. Isolation of the product generally is achieved by simply removing the reaction solvent by evaporation under reduced pressure. Further purification, if needed, can be accomplished by crystallization, chromatography or similar conventional methods.

Thus, another aspect of the invention is that it provides (dialkoxy)ethyl thiazolylureas of the formula (II):

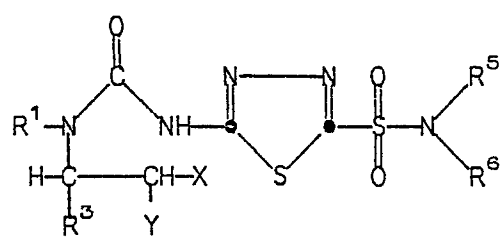

(II)

wherein:

$R^1$ is hydrogen or $C_1$-$C_6$ alkyl;

$R^3$ is hydrogen or $C_1$-$C_6$ alkyl;

$R^5$ is hydrogen or $C_1$-$C_6$ alkyl, and

$R^6$ is hydrogen, $C_1$-$C_6$ alkyl, $C_2$-$C_6$ alkenyl, $C_2$-$C_6$ alkynyl, $C_1$-$C_6$ alkoxy, or

$R^5$ and $R^6$ taken together with the nitrogen atom to which they are attached form pyrrolidino, piperidino, or morpholino, and

X and Y independently are $C_1$-$C_6$ alkoxy.

The preparation of typical thiadiazolylurea intermediates and thiadiazolylimidazolidinone derivatives is illustrated by the following working examples. The examples are representative only and are not intended to be limiting in any respect and should not be so construed.

Example 1

3-(5-Dimethylaminosulfonyl-1,3,4-thiadiazol-2-yl)-4-hydroxy-1-methyl-2-imidazolidinone

A. Preparation of 2-(N-phenoxycarbonylamino)-1,3,4-thiadiazol-5-thiol.

To a stirred solution of 25 g (0.19 moles) of 2-amino-1,3,4-thiadiazol-5-thiol in 75 ml of pyridine were added dropwise over 30 minutes 32.8 g (0.21 moles) of phenyl chloroformate. Following complete addition the reaction mixture was warmed to room temperature and stirred for 16 hours. The reaction mixture was then poured into ice and the pH was adjusted to 2.0 by the addition of hydrochloric acid. The solid which precipitated was collected by filtration and washed with water and air dried to provide 48 g of 2-(N-phenoxycarbonylamino)-1,3,4-thiadiazol-5-thiol. Melting point 203-207°C.

B. Preparation of 2-(N-phenoxycarbonylamino)-5-dimethylaminosulfonyl-1,3,4-thiadiazole.

To a cold (5-10°C.) stirred suspension of 2-(N-phenoxycarbonylamino)-1,3,4-thiadiazol-5-thiol in 60 ml. of 12N hydrochloric acid and 200 ml. of water was bubbled chlorine until chlorine was no longer absorbed. The precipitate that formed was filtered and

washed with water to provide 51 g of 2-(N-phenoxycarbonylamino)-5-chlorosulfonyl-1,3,4-thiadiazole. mp 166-169°C. The product thus formed was added portionwise over 30 minutes to a stirred mixture of 60 ml of 25% aqueous dimethylamine and 30 ml of water. The reaction mixture was stirred overnight at room temperature following complete addition. The reaction mixture next was filtered and the precipitate was washed with water and air dried to give 17 g of 2-(N-phenoxycarbonylamino)-5-dimethylaminosulfonyl-1,3,4-thiadiazole. mp 155-159°C.

C. Preparation of 1-(5-Dimethylaminosulfonyl-1,3,4-thiadiazol-2-yl)-3-(2,2-dimethoxyethyl)-3-methylurea.

A suspension of 25 g (0.076 moles) of 2-(N-phenoxycarbonylamino)-5-dimethylaminosulfonyl-1,3,4-thiadiazole in 100 ml of benzene containing 10 g (0.084 moles) of N-methyl-(2,2-dimethoxy)ethylamine was heated at reflux for 12 hours. The reaction mixture was cooled to room temperature and the solvent was removed by evaporation under reduced pressure to provide 23 g of a dark oil. The oil was shown to be a mixture of 1-(5-dimethylaminosulfonyl-1,3,4-thiadiazol-2-yl)-3-(2,2-dimethoxyethyl)-3-methylurea and phenol.

D. A suspension of the oil in 160 ml of water containing 4 ml. of concentrated hydrochloric acid was heated at reflux for 30 minutes. The reaction mixture was then cooled to room temperature and stirred for 12 hours. The precipitate that had formed was

filtered and washed with water and then was crystallized from ethyl acetate and recrystallized from ethanol to provide 7.4 g of 3-(5-dimethylaminosulfonyl-1,3,4-thiadiazol-2-yl)-4-hydroxy-1-methyl-2-imidazolidinone. mp 194-196°C.

Example 2

3-(5-Diethylaminosulfonyl-1,3,4-thiadiazol-2-yl)-4-hydroxy-1-methyl-2-imidazolidinone

A. Following the general procedures set forth in Example 1, 3.9 g (0.025 moles) of phenylchloroformate were added dropwise over 30 minutes to a stirred solution of 5.4 g (0.023 moles) of 2-amino-5-diethylaminosulfonyl-1,3,4-thiadiazole in 40 ml of pyridine. The reaction mixture was stirred at about 10°C. during the addition of the phenylchloroformate, and following the addition the reaction mixture was warmed to about 20°C and stirred at that temperature for about 3 hours. The reaction mixture was then added to 50 g of ice and diluted with one normal hydrochloric acid to pH 2.0. The precipitated solid was collected by filtration and washed with water and air dried to give 10.8 g of 2-(N-phenoxycarbonylamino)-5-diethylaminosulfonyl-1,3,4-thiadiazole. mp 126-130°C.

B. A suspension of 10.8 g of the thiadiazole-carbamate prepared as described above in 100 ml of toluene was stirred while 3.9 g (0.033 mole) of N-methyl-(2,2-dimethoxy)ethylamine were added dropwise over 20 minutes. The reaction mixture was then heated at 80°C for 2 hours and then cooled to room temperature and the solvent was removed by evaporation under reduced pressure to provide 6.3 g of the product as an oil. The oil solidified on standing and the solid was crystallized from ethyl acetate and hexane to give 1-(5-diethylaminosulfonyl-1,3,4-thiadiazol-2-yl)-3-(2,2-dimethoxyethyl)-3-methylurea. mp 110-112°C.

Analysis calculated for $C_{12}H_{23}N_5O_5S_2$
Theory: C, 37.78; H, 6.08; N, 18.36.
Found: C, 37.76; H, 5.82; N, 18.12.

C. A solution of 4.3 g of the thiadiazolylurea thus prepared was suspended in 80 ml of water containing 2 ml of concentrated hydrochloric acid. The reaction mixture was heated at reflux for 45 minutes and then cooled to room temperature and added to 50 g of ice. The product crystallized out of the aqueous mixture and was collected by filtration and recrystallized from ethyl acetate and hexane to give 2.0 g of 3-(5-diethylaminosulfonyl-1,3,4-thiadiazol-2-yl)-4-hydroxy-1-methyl-2-imidazolidinone. mp 161-163°C.

Analysis calculated for $C_{10}H_{17}N_5O_4S_2$
Theory: C, 35.82; H, 5.07; N, 20.90.
Found: C, 36.05; H, 4.85; N, 20.94.

## Example 3

3-(5-N-methyl-N-ethylaminosulfonyl-1,3,4-thiadiazol-2-yl)-4-hydroxy-1-methyl-2-imidazolidinone

A. Following the general procedures set forth in Examples 1 and 2, 11.5 g (0.033 moles) of 2-phenoxycarbonylamino-5-(N-methyl-N-ethylaminosulfonyl)-1,3,4-thiadiazole were reacted with 4.3 g (0.036 moles) of N-methyl-(2,2-dimethoxy)ethylamine in 100 ml

of benzene to give 14 g of 1-(5-N-methyl-N-ethylaminosulfonyl-1,3,4-thiadiazol-2-yl)-3-(2,2-dimethoxyethyl)-3-methylurea.

B. The urea thus formed was reacted with hydrochloric acid in water to provide 1.72 g of 3-(5-N-methyl-N-ethylaminosulfonyl-1,3,4-thiadiazol-2-yl)-4-hydroxy-1-methyl-2-imidazolidinone. mp 130-132°C.

Analysis calculated for $C_9H_{15}N_5O_4S_2$

Theory: C, 33.64; H, 4.67; N, 21.81.

Found: C, 33.63; H, 4.51; N, 21.55.

Example 4

3-(5-N-methyl-N-propylaminosulfonyl-1,3,4-thiadiazol-2-yl)-4-hydroxy-1-methyl-2-imidazolidinone

A. Following the general procedure set forth in Examples 1 and 2, 14.3 g (0.04 moles) of 2-phenoxycarbonylamino-5-(N-methyl-N-propylaminosulfonyl)-1,3,4-thiadiazole were reacted with 5.2 g (0.044 moles) of N-methyl-(2,2-dimethoxy)ethylamine in refluxing benzene to give 13.2 g of 3-(5-N-methyl-N-propylaminosulfonyl-1,3,4-thiadiazol-2-yl)-1-(2,2-dimethoxyethyl)-1-methylurea as an oil.

B. The thiadiazolylurea thus prepared, 13.2 g (0.035 moles), was reacted with 2 ml of concentrated hydrochloric acid and 80 ml of water at about 100°C. for 30 minutes. The reaction mixture was cooled to room temperature and added to ice. The product was extracted into ethyl acetate and the organic extracts were combined, washed with water, dried and the solvent was removed by evaporation under reduced pressure to

provide, after recrystallization from ethyl acetate and hexane, 1.45 g of 3-(5-N-methyl-N-propylaminosulfonyl-1,3,4-thiadiazol-2-yl)-4-hydroxy-1-methyl-2-imidazolidinone. mp 126-128°C.

Analysis calculated for $C_{10}H_{17}N_5O_4S_2$

Theory: C, 35.81; H, 5.11; N, 20.88.

Found: C, 35.99; H, 4.93; N, 20.80.

Example 5

3-[5-N-methyl-N-(*n*-butyl)aminosulfonyl-1,3,4-thiadiazol-2-yl]-4-hydroxy-1-methyl-2-imidazolidinone

A. 5-Amino-1,3,4-thiadiazol-2-thiol was reacted with chlorine in an aqueous solution of hydrochloric acid to provide 2-amino-5-chlorosulfonyl-1,3,4-thiadiazole.

B. The product of step A was reacted with N-methyl-*n*-butylamine in the presence of triethylamine in tetrahydrofuran to provide 2-amino-5-[N-methyl-N-(*n*-butyl)aminosulfonyl]-1,3,4-thiadiazole.

C. Reaction of the compound produced in step B with phenylchloroformate according to the procedure of Example 1 provided 2-phenoxycarbonylamino-5-[N-methyl-N-(n-butyl)aminosulfonyl]-1,3,4-thiadiazole.

D. To a stirred solution of 13.7 g (0.037 mole) of 2-phenoxycarbonylamino-5-[N-methyl-N-(n-butyl-aminosulfonyl]-1,3,4-thiadiazole and 100 ml of benzene were added dropwise over 30 minutes 4.9 g (0.04 mole) of N-methyl-(2,2-dimethoxy)ethylamine. The reaction mixture was heated at reflux for 3 hours and then cooled to room temperature. The solid precipitate was

collected by filtration to provide 6.9 g of 1-[5-N-methyl-N-(n-butyl)aminosulfonyl-1,3,4-thiadiazol-2-yl]-3-(2,2-dimethoxy)ethyl-3-methylurea. A sample which was recrystallized from ethanol and water melted at mp 95-97°C.

Analysis calculated for $C_{13}H_{25}N_5O_5S_2$
Theory: C, 39.48; H, 6.37; N, 17.71.
Found: C, 39.72; H, 6.13; N, 17.50.

E. A solution of the thiadiazolylurea in 80 ml of water containing 2 ml of concentrated hydrochloric acid was heated at reflux for 45 minutes. The reaction mixture was then cooled and added to 50 g of ice. The aqueous mixture was extracted several times with ethyl acetate, and the organic extracts were combined, washed with water, dried and the solvent was removed by evaporation under reduced pressure to provide the product as an oil. The oil was crystallized from ethyl acetate and hexane to afford 1.5 g of 3-[5-N-methyl-N-(n-butyl)aminosulfonyl-1,3,4-thiadiazol-2-yl]-4-hydroxy-1-methyl-2-imidazolidinone. mp 105-107°C.

Analysis calculated for $C_{11}H_{19}N_5O_4S_2$
Theory: C, 37.81; H, 5.48; N, 20.04.
Found: C, 37.52; H, 5.28; N, 19.84.

Example 6

3-[5-N-methyl-N-(2-propynyl)aminosulfonyl-1,3,4-thiadiazol-2-yl]-4-hydroxy-1-methyl-2-imidazolidinone

A. N-methylpropargylamine was reacted with 2-amino-5-chlorosulfonyl-1,3,4-thiadiazole to provide

2-amino-5-[N-methyl-N-(2-propynyl)aminosulfonyl]-1,3,4-thiadiazole.

B. The aminothiadiazole derivative produced in step A was reacted with phenylchloroformate to give 2-phenoxycarbonylamino-5-[N-methyl-N-(2-propynyl)aminosulfonyl]-1,3,4-thiadiazole.

C. A solution of 12 g (0.034 mole) of 2-phenoxycarbonylamino-5-[N-methyl-N-(2-propynyl)aminosulfonyl]-1,3,4-thiadiazole in 100 ml of benzene containing 4.4 g (0.037 mole) of N-methyl-(2,2-dimethoxy)ethylamine was heated at reflux for 3 hours and then cooled to room temperature. The reaction solvent was removed by evaporation under reduced pressure to provide 11.0 g of 1-[5-N-methyl-N-(2-propynyl)aminosulfonyl-1,3,4-thiadiazol-2-yl]-3-(2,2-dimethoxyethyl)-3-methylurea. A portion of the product was recrystallized from ethanol and water and melted at 107-109°C.

Analysis calculated for $C_{12}H_{19}N_5O_5S_2$

Theory: C, 38.19; H, 5.07; N, 18.56.

Found: C, 37.98; H, 4.86, N, 18.31.

D. Cyclization of 8.0 g of this thiadiazolylurea derivative by reaction with hydrochloric acid and water according to the procedure outlined above in Example 1 provided 3.6 g of 3-[5-N-methyl-N-(2-propynyl)-aminosulfonyl-1,3,4-thiadiazol-2-yl]-4-hydroxy-1-methyl-2-imidazolidinone. mp 146-148°C.

Analysis calculated for $C_{10}H_{13}N_5O_4S_2$

Theory: C, 36.25; H, 3.95; N, 21.13.

Found: C, 36.08; H, 3.69; N, 20.93.

## Example 7

3-(5-N-methyl-N-methoxyaminosulfonyl-1,3,4-thiadiazol-2-yl)-4-hydroxy-1-methyl-2-imidazolidinone

A. By following the general procedure outlined above, N-methylmethoxyamine was reacted with 2-amino-5-chlorosulfonyl-1,3,4-thiadiazole to give 2-amino-5-N-methyl-N-methoxyaminosulfonyl-1,3,4-thiadiazole.

B. The product of step A was reacted with phenylchloroformate to give the corresponding phenylcarbamate derivative.

C. The product of step B was then reacted with N-methyl-(2,2-dimethoxy)ethylamine to provide 1-(5-N-methyl-N-methoxyaminosulfonyl-1,3,4-thiadiazol-2-yl)-3-(2,2-dimethoxyethyl)-3-methylurea. mp 134-136°C.

Analysis calculated for $C_{10}H_{19}N_5O_6S_2$

Theory: C, 32.51; H, 5.18; N, 18.96.

Found: C, 32.57; H, 5.16; N, 19.04.

D. Cyclization of 4.0 g of the thiadiazolylurea derivative produced in step C by reaction with hydrochloric acid and water provided, following crystallization from ethyl acetate, 2.03 g of 3-(5-N-methyl-N-methoxyaminosulfonyl-1,3,4-thiadiazol-2-yl)-4-hydroxy-1-methyl-2-imidazolidinone. mp 178-180°C.

Analysis calculated for $C_8H_{13}N_5O_5S_2$

Theory: C, 29.72; H, 4.05; N, 21.66.

Found: C, 29.75; H, 4.15; N, 21.86.

Example 8

3-(5-Dimethylaminosulfonyl-1,3,4-thiadiazol-2-yl)-1-methyl-2-imidazolinone

A solution of 3 g of 3-(5-dimethylaminosulfonyl-1,3,4-thiadiazol-2-yl)-4-hydroxy-1-methyl-2-imidazolidinone (prepared as described in Example 1) in 20 g of acetyl chloride was stirred at 24°C for 72 hours. The reaction mixture was then concentrated by evaporation of the acetyl chloride under reduced pressure and the residue was crystallized from methanol and again from ethyl acetate to provide 0.5 g of 3-(5-dimethylaminosulfonyl-1,3,4-thiadiazol-2-yl)-1-methyl-2-imidazolinone. mp 192-195°C.

Analysis calculated for $C_8H_{11}N_5O_3S_2$

Theory: C, 33.21; H, 3.83; N, 24.21.

Found: C, 33.46; H, 3.81; N, 24.00.

Example 9

3-(5-Dimethylaminosulfonyl-1,3,4-thiadiazol-2-yl)-4-acetoxy-1-methyl-2-imidazolidinone

A solution of 1 g (0.003 mole) of 3-(5-dimethylaminosulfonyl-1,3,4-thiadiazol-2-yl)-4-hydroxy-1-methyl-2-imidazolidinone (from Example 1) in 10 ml of dichloromethane was cooled to 0°C and stirred. To the cold stirred reaction mixture were added 0.31 g (0.004 mole) of pyridine followed by the dropwise addition of 0.31 g (0.004 mole) of acetyl chloride over 20 minutes. The reaction mixture was stirred at 0°C for 5 hours following complete addition. The mixture was then filtered and the filtrate was reduced in volume by

evaporation of the solvent under reduced pressure to give an oil. The oil was added to 50 ml of water and the aqueous mixture was diluted with 1N hydrochloric acid to pH 7. The aqueous mixture was extracted several times with ethyl acetate and the extracts were combined, washed with water, dried and the solvent was removed by evaporation under reduced pressure. The product was crystallized once from ethanol and again from ethyl acetate to afford 0.4 g of 3-(5-dimethylaminosulfonyl-1,3,4-thiadiazol-2-yl)-4-acetoxy-1-methyl-2-imidazolidinone. mp 163-165°C. Analysis calculated for $C_{10}H_{15}N_5O_5S_2$

Theory: C, 34.38; H, 4.33; N, 20.05.
Found: C, 34.66; H, 4.35; N, 20.04.

## Example 10

3-(5-Dimethylaminosulfonyl-1,3,4-thiadiazol-2-yl)-4-methoxy-1-methyl-2-imidazolidinone

A solution of 2.0 g of 3-(5-dimethylaminosulfonyl-1,3,4-thiadiazol-2-yl)-4-hydroxy-1-methyl-2-imidazolidinone (prepared according to the method of Example 1) in 15 ml of methanol was stirred at room temperature while 5 drops of concentrated sulfuric acid were added. The reaction mixture was stirred at room temperature for 2 hours and then heated to reflux for 12 hours. The mixture was cooled to room temperature and then filtered. The precipitate was washed with fresh methanol. The filtrate was concentrated to dryness by evaporation of the solvent under reduced pressure and the solid thus formed was crystallized

from ethanol to give 0.4 g of 3-(5-dimethylaminosulfonyl-1,3,4-thiadiazol-2-yl)-4-methoxy-1-methyl-2-imidazolidinone. mp 129-131°C.

Analysis calculated for $C_9H_{15}N_5O_4S_2$

Theory: C, 33.64; H, 4.70; N, 21.79.

Found: C, 33.54; H, 4.81; N, 21.56.

Example 11

3-(5-Dimethylaminosulfonyl-1,3,4-thiadiazol-2-yl)-4-hydroxy-2-imidazolidinone

A. To a stirred solution of 12.0 g (0.036 mole) of 2-phenoxycarbonylamino-5-dimethylaminosulfonyl-1,3,4-thiadiazole in 100 ml of toluene were added dropwise over 20 minutes 4.2 g (0.040 mole) of 2,2-dimethoxyethylamine. The reaction mixture was heated at 80° for 2 hours and then cooled and concentrated by removal of the reaction solvent by evaporation under reduced pressure. The product thus formed was an oil that solidified on standing. Recrystallization from ethanol afforded 4.7 g of 1-(5-dimethylaminosulfonyl-1,3,4-thiadiazol-2-yl)-3-(2,2-dimethoxyethyl)urea. mp 172-174°C.

Analysis calculated for $C_9H_{17}N_5O_5S_2$

Theory: C, 31.85; H, 5.05; N, 20.64.

Found: C, 32.14; H, 5.18; N, 20.78.

B. A solution of 4 g of 1-(5-dimethylaminosulfonyl)-1,3,4-thiadiazol-2-yl)-3-(2,2-dimethoxyethyl)urea in 80 ml of water containing 2 ml of concentrated hydrochloric acid was stirred at reflux for 30 minutes. The mixture was then cooled and the product was collected by filtration. The solid product was washed with fresh water and air dried to give 2.2 g of 3-(5-dimethylaminosulfonyl-1,3,4-thiadiazol-2-yl)-4-hydroxy-2-imidazolidinone. mp 204-206°C.

Analysis calculated for $C_7H_{11}N_5O_4S_2$

Theory: C, 28.66; H, 3.78; N, 23.88.

Found: C, 28.53; H, 3.67; N, 23.68.

Example 12

3-(5-Dimethylaminosulfonyl-1,3,4-thiadiazol-2-yl)-4-hydroxy-1-phenyl-2-imidazolidinone

A. To a stirred solution of 5.0 g of 2-phenoxycarbonylamino-5-dimethylaminosulfonyl-1,3,4-thiadiazole in 30 ml of tetrahydrofuran were added dropwise over 30 minutes 3.6 g of N-phenyl-N-(2,2-dimethoxyethyl)amine. Following complete addition, 1 ml of pyridine was added to the reaction mixture and the mixture was heated at reflux for 16 hours. The reaction mixture was then cooled to room temperature and concentrated in volume by removal of the reaction solvent by evaporation under reduced pressure. The residue was poured into 20 ml of ice water and the pH of the aqueous mixture was adjusted to 5 by the addition of dilute hydrochloric acid. The aqueous acid mixture was extracted several times with ethyl acetate and the extracts were combined, washed with water and dried. Removal of the solvent by evaporation under reduced pressure provided the product as an oil. The oil was purified by column chromatography over silica gel, eluting with diethyl ether saturated with water.

Removal of the solvent from the appropriate fractions afforded 2.2 g of 1-(5-dimethylaminosulfonyl-1,3,4-thiadiazol-2-yl)-3-phenyl-3-(2,2-dimethoxyethyl)urea. mp 59-61°C.

Analysis calculated for $C_{15}H_{21}N_5O_5S_2$
Theory: C, 43.36; H, 5.09; N, 16.86.
Found: C, 43.59; H, 5.02; N, 16.96.

B. The thiadiazolylurea thus formed (1.8 g) was cyclized by reaction with 1 ml of concentrated hydrochloric acid in 40 ml of water. The reaction mixture was heated at reflux for 30 minutes and then cooled and extracted with ethyl acetate. The combined organic extracts were washed with water, dried, and the solvent was removed by evaporation under reduced pressure. The solid that was formed was crystallized from ethanol and water and again from ethyl acetate to afford 180 mg of 3-(5-dimethylaminosulfonyl-1,3,4-thiadiazol-2-yl)-4-hydroxy-1-phenyl-2-imidazolidinone. mp 179-181°C.

Analysis calculated for $C_{13}H_{15}N_5O_4S_2$
Theory: C, 42.28; H, 4.07; N, 18.97.
Found: C, 42.11; H, 3.85; N, 18.82.

## Example 13

3-(5-Dimethylaminosulfonyl-1,3,4-thiadiazol-2-yl)-4-methylaminocarbonyloxy-1-methyl-2-imidazolidinone

To a stirred suspension of 2.0 g (0.007 mole) of 3-(5-dimethylaminosulfonyl-1,3,4-thiadiazol-2-yl)-4-hydroxy-1-methyl-2-imidazolidinone (prepared as

described in Example 1) in 15 ml of toluene were added dropwise 0.49 ml (0.008 mole) of methyl isocyanate. Following complete addition, the reaction mixture was heated at reflux for 16 hours. The reaction mixture was filtered while hot, and the filtrate was then cooled to room temperature. The solvent was removed by evaporation under reduced pressure to provide the product as a solid. The solid was recrystallized from ethanol to afford 0.66 g of 3-(5-dimethylaminosulfonyl-1,3,4-thiadiazol-2-yl)-4-methylaminocarbonyloxy-1-methyl-2-imidazolidinone. mp 190-192°C. Analysis calculated for $C_{10}H_{16}N_6O_5S_2$

Theory: C, 32.96; H, 4.43; N, 23.06.
Found: C, 32.90; H, 4.55; N, 22.86.

Example 14

Following the general procedure of Example 13 1 g of 3-(5-dimethylaminosulfonyl-1,3,4-thiadiazol-2-yl)-4-hydroxy-1-methyl-2-imidazolidinone from Example 1 was reacted with 0.48 g of phenyl isocyanate to provide, following crystallization from acetone, 0.3 g of 3-(5-dimethylaminosulfonyl-1,3,4-thiadiazol-2-yl)-4-phenylaminocarbonyloxy-1-methyl-2-imidazolidinone. mp 221-223°C. Analysis calculated for $C_{15}H_{18}N_6O_5S_2$

Theory: C, 42.25; H, 4.25; N, 19.71.
Found: C, 42.02; H, 4.08; N, 19.51.

## Example 15

3-(5-Dimethylaminosulfonyl-1,3,4-thiadiazol-2-yl)-4-dimethylamino-1-methyl-2-imidazolidinone

Two grams of 3-(5-dimethylaminosulfonyl-1,3,4-thiadiazol-2-yl)-4-hydroxy-1-methyl-2-imidazolidinone (prepared as described in Example 1) were suspended in 10 ml of THF. To the stirred reaction mixture were added 1.14 g of dimethylamine hydrochloride and 1.42 g of triethylamine. The reaction mixture was heated at 60° for 7 hours and then cooled to room temperature and stirred for an additional 16 hours. Thin layer chromatographic analysis indicated that the reaction mixture was comprised of approximately 50% starting material. The reaction mixture was therefore heated at 60°C. for an additional seven and one-half hours, and then stirred for 12 hours at room temperature. The solvent was removed from the reaction mixture by evaporation under reduced pressure to provide the product as an oil. The oil was dissolved in 100 ml of ethyl acetate and washed with water, dried, and the solvent was removed by evaporation. The product thus formed was purified by column chromatography over silica gel, using ethyl acetate as eluant. Evaporation of the solvent from the appropriate fractions provided 1.25 g of 3-(5-dimethylaminosulfonyl-1,3,4-thiadiazol-2-yl)-4-dimethylamino-1-methyl-2-imidazolidinone. mp 98-100°C. Analysis calculated for $C_{10}H_{18}N_6O_3S_2$

Theory: C, 35.92; H, 5.43; N, 25.13.
Found: C, 35.66; H, 5.22; N, 24.96.

## Example 16

3-(5-Dimethylaminosulfonyl-1,3,4-thiadiazol-2-yl)-4-hydroxy-1-methyl-2-imidazolidinone

This Example is a preferred procedure for the synthesis of the compound of Example 1.

A. Into a stirred suspension of 50 g (0.38 mole) of 2-amino-1,3,4-thiadiazol-5-thiol in 200 ml of water containing 60 ml of concentrated hydrochloric acid was bubbled chlorine until the suspension became saturated and chlorine was no longer absorbed. The reaction mixture was then filtered and the brown solid was identified as 56 g of 2-amino-5-chlorosulfonyl-1,3,4-thiadiazole. mp 127-130°C.

B. The 2-amino-5-chlorosulfonyl-1,3,4-thiadiazole was suspended in 200 ml of water and stirred at 0-10°C while 140 ml of 25% (w/w) aqueous dimethylamine were added dropwise over thirty minutes. The reaction mixture was warmed to room temperature following the addition, and stirring was continued for one hour. The solid was collected by filtration and air dried to give 46 g of 2-amino-5-dimethylaminosulfonyl-1,3,4-thiadiazole. mp 152-155°C.

C. To a cold (0-5°C) stirred solution of 20.0 g (0.096 mole) of 2-amino-5-dimethylaminosulfonyl-1,3,4-thiadiazole in 75 ml of pyridine were added 17.2 g of phenyl chloroformate dropwise over thirty minutes. The reaction mixture was then warmed to room temperature and stirred for an additional two and one-half hours. The reaction mixture was next added to 200 g of ice water and acidified to pH 2 by the addition of hydrochloric acid. The precipitated solid was

collected by filtration, washed with water, dried, and identified as 31.3 g of 2-(N-phenoxycarbonylamino)-5-dimethylaminosulfonyl-1,3,4-thiadiazole. mp 186-190°C.

D. The above procedure was repeated several times to produce 1012 g of 2-(N-phenoxycarbonylamino)-5-dimethylaminosulfonyl-1,3,4-thiadiazole. This entire lot was then suspended in 2800 ml of benzene, and the suspension was stirred at room temperature while 405 g of N-methyl-(2,2-dimethoxy)ethylamine were added dropwise over two hours. Following the addition, the reaction mixture was heated at reflux for two hours. The reaction mixture was next cooled to room temperature and the solvent was removed by evaporation under reduced pressure to give 1010 g of 1-(5-dimethylaminosulfonyl-1,3,4-thiadiazol-2-yl)-3-(2,2-dimethoxyethyl)-3-methylurea (containing slight amount of phenol impurity). mp 120-124°C.

E. A suspension of 1010 g of the urea thus prepared in 6000 ml of water containing 150 ml of concentrated hydrochloric acid was heated at reflux for thirty minutes. The reaction mixture was then cooled and filtered. The filter cake was washed with fresh water and then air dried. The solid was next slurried in 8 liters of ethanol, filtered, and then washed with diethyl ether and air dried to give 691 g of 3-(5-dimethylaminosulfonyl-1,3,4-thiadiazol-2-yl)-4-hydroxy-1-methyl-2-imidazolidinone. mp 194-196°C.

Analysis calculated for $C_8H_{13}N_5O_4S_2$

Theory: C, 31.26; H, 4.26; N, 22.79.

Found: C, 31.13; H, 4.01; N, 22.56.

The thiadiazolylimidazolidinone derivatives provided by this invention exhibit both terrestrial and aquatic herbicidal and plant growth regulator activity, and accordingly are useful in the control and elimination of unwanted vegetative growth. The compounds are non-toxic to a number of beneficial terrestrial plants, including cotton, corn, soybean, wheat, rice, barley, oats, peanuts and related crops raised for both human and domestic animal consumption.

The herbicides of the invention are effective terrestrially in both pre-emergent and early post-emergent control of a wide variety of grasses and broadleaf weeds. Commonly encountered unwanted terrestrial vegetation which is subject to control with the herbicidal thiadiazolylimidazolidinones and thiadiazolylimidazolinones of this invention include annuals such as pigweed, lambsquarter, wild oats, velvetleaf, ryegrass, goose grass, crabgrass, foxtail, chickweed, barnyardgrass, smartweed, knotweed, cocklebur, kochia, medic, ragweed, hemp nettle, spurge, jungle rice, morningglory, ducksalad, cheatgrass, fall panicum, jimsonweed, watergrass, spangletop, and the like. Biennials subject to control include wild carrot, wild barley, burdock, bull thistle, houndstongue, and purple star thistle. Other common weeds which can be controlled with the herbicides of this invention include perennial ryegrass, quackgrass, Johnson grass, sheep sorrell, nutgrass, field chickweed, dandelion, Russian Knotweed, horsetail, sesbania, cattail, wintercress, nutsedge, milkweed, sicklepod, black nightshade, and related weeds.

The pre-emergent and post-emergent terrestrial herbicidal activity of several thiadiazolylimidazolidinone derivatives of this invention has been determined in standard greenhouse experiments. Such experiments were carried out by first formulating a test compound for convenient soil surface or overtop foliar spray application. The test compound was formulated by dissolving 0.2 percent by weight in a solution containing 4.0 percent by weight acetone, 4.0 percent by weight ethanol, and 91.7 percent by weight of deionized water, and 0.1 percent by weight of a commercial surfactant, for example Toximul R and S, which are blends of anionic and nonionic surface active agents (Stepan Chemical Co. Northfield, Illinois, 60093). The solution containing the test compound was then serially diluted with deionized water to the appropriate volume containing the desired concentration of test compound. The formulated compounds were applied pre-emergence and/or post-emergence to metal greenhouse flats filled with soil and seeded to test plants. The compounds were evaluated at several application rates, initially at 15 pounds per acre (16.8 kg/ha) and at lower rates until compound activity diminished. Pre-emergence applications were made to the soil surface of the seeded flats. Post-emergence applications were made to the foliage of the various plant species approximately twelve days after seeding. All treated flats were maintained in a greenhouse following treatment. Evaluations of compound activity were made in the form of plant injury rating on a scale of 1 to 5. The rating of "1" refers to no plant injury; "2" slight

Table 15

Plant Injury Ratings for 3-(5-dimethylaminosulfonyl-1,3,4-thiadiazol-2-yl)-4-dimethylamino-1-methyl-2-imidazolidinone of Example 15

PLANT SPECIES

| appln. rate lbs/A | (kg/ha) | Pre-emergence Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Post-emergence Corn | Large Crabgrass | Pigweed | Foxtail | Velvetleaf | Morningglory | Zinnia |
|---|---|---|---|---|---|---|---|---|---|---|---|---|---|---|---|---|---|---|---|---|---|---|---|---|---|---|---|---|
| 8 | (8.96) | 4 | - | - | - | - | - | - | - | - | - | - | 5 | - | 5 | 4 | - | 4 | - | 5 | 5 | 2 | 3 | 4 | 4 | 4 | 4 | 4 |
| 4 | (4.5) | 1 | 3 | 2 | 3 | 5 | 5 | 2 | 4 | 3 | 4 | 4 | 3 | 2 | 4 | 5 | 4 | 5 | 4 | 3 | 5 | 5 | 4 | 5 | 5 | 4 | 3 | 5 |
| 2 | (2.7) | 1 | 1 | 1 | 2 | 5 | 4 | 2 | 3 | 3 | 3 | 5 | 4 | 2 | 4 | 4 | 3 | 3 | - | 2 | 5 | 3 | 4 | 5 | 5 | 4 | 3 | 5 |
| 1 | (1.12) | 1 | 1 | 1 | 2 | 3 | 2 | 1 | 1 | 1 | 2 | 2 | 3 | 2 | 3 | 3 | 2 | 3 | - | 1 | 3 | 2 | 3 | 5 | 4 | 3 | 3 | 4 |

## Table 14

Plant Injury Ratings for 3-(5-dimethylaminosulfonyl-1,3,4-thiadiazol-2-yl)-4-phenylaminocarbonyloxy-1-methyl-2-imidazolidinone of Example 14

PLANT SPECIES

| | | Pre-emergence | | | | | | | | | | | | | | | | | | | | Post-emergence | | | | | | |
|---|---|---|---|---|---|---|---|---|---|---|---|---|---|---|---|---|---|---|---|---|---|---|---|---|---|---|---|---|
| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Corn | Large Crabgrass | Pigweed | Foxtail | Velvetleaf | Morningglory | Zinnia |
| 8 | (8.96) | 1 | - | - | - | - | - | - | - | - | - | - | 1 | - | 1 | 1 | - | 1 | - | 1 | 1 | 1 | 1 | 1 | 2 | 1 | 2 | 2 |

## Table 13

Plant Injury Ratings for 3-(5-dimethylaminosulfonyl-1,3,4-thiadiazol-2-yl)-4-methylaminocarbonyloxy-1-methyl-2-imidazolidinone of Example 13

| | | PLANT SPECIES | | | | | | | | | | | | | | | | | | | | | | | | | |
|---|---|---|---|---|---|---|---|---|---|---|---|---|---|---|---|---|---|---|---|---|---|---|---|---|---|---|---|
| | | Pre-emergence | | | | | | | | | | | | | | | | | | | | Post-emergence | | | | | | |
| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Corn | Large Crabgrass | Pigweed | Foxtail | Velvetleaf | Morningglory | Zinnia |
| 8 | (8.96) | 2 | - | - | | - | - | - | - | - | - | - | 5 | - | 5 | 5 | - | 5 | - | 3 | 5 | 2 | 3 | 5 | 4 | 3 | 3 | 4 |
| 4 | (4.5) | 1 | 1 | 1 | 3 | 5 | 3 | 4 | 4 | 4 | 5 | 5 | 4 | 4 | 5 | 4 | - | 3 | 4 | 4 | 5 | 1 | 3 | 5 | 3 | 1 | 3 | 4 |
| 2 | (2.7) | 1 | 1 | 1 | 1 | 1 | 1 | 1 | 1 | 2 | 2 | 2 | 3 | 2 | 4 | 3 | - | 1 | 1 | 3 | 4 | 1 | 3 | 4 | 2 | 1 | 2 | 2 |
| 1 | (1.12) | 1 | 1 | 1 | 1 | 1 | 1 | 1 | 1 | 1 | 1 | 1 | 1 | 1 | 2 | 1 | - | 1 | 1 | 1 | 1 | 1 | 3 | 3 | 2 | 1 | 1 | 2 |

Table 12

Plant Injury Ratings for 3-(5-dimethylaminosulfonyl-1,3,4-thiadiazol-2-yl)-4-hydroxy-1-phenyl-2-imidazolidinone from Example 12

PLANT SPECIES

| appln. rate lbs/A | (kg/ha) | Pre-emergence Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Post-emergence Corn | Large Crabgrass | Pigweed | Foxtail | Velvetleaf | Morningglory | Zinnia |
|---|---|---|---|---|---|---|---|---|---|---|---|---|---|---|---|---|---|---|---|---|---|---|---|---|---|---|---|---|
| 8 | (8.96) | 1 | - | - | - | - | - | - | - | - | - | - | 1 | - | 1 | 1 | - | 1 | - | 1 | 1 | 1 | 1 | 1 | 1 | 1 | 1 | 1 |

Table 11

Plant Injury Ratings for 3-(5-dimethylaminosulfonyl-1,3,4-thiadiazol-2-yl)-4-hydroxy-2-imidazolidinone from Example 11

PLANT SPECIES

| appln. rate lbs/A | (kg/ha) | Pre-emergence | | | | | | | | | | | | | | | | | | | | Post-emergence | | | | | | | |
|---|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Tomato | Corn | Large Crabgrass | Pigweed | Foxtail | Velvetleaf | Morningglory | Zinnia |
| 15 | (16.8) | - | - | - | - | - | - | - | - | 1 | - | - | 1 | - | 2 | - | - | - | - | - | - | 2 | - | 1 | 4 | - | - | - | - |
| 8 | (8.96) | 1 | - | - | - | - | - | - | - | - | - | - | 2 | - | 2 | 1 | - | 1 | - | 1 | 1 | | 1 | 1 | 2 | 1 | 1 | 1 | 1 |

Table 10

Plant Injury Ratings for 3-(5-dimethylaminosulfonyl-1,3,4-thiadiazol-2-yl)-4-methoxy-1-methyl-2-imidazolidinone from Example 10

PLANT SPECIES

Pre-emergence

| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia |
|---|---|---|---|---|---|---|---|---|---|---|---|---|---|---|---|---|---|---|---|---|---|
| 2 | (2.71) | 1 | 1 | 1 | 1 | 4 | 3 | 3 | 4 | 3 | 4 | 5 | 4 | 4 | 5 | 5 | - | 3 | 4 | 4 | 5 |
| 1 | (1.12) | 1 | 1 | 1 | 1 | 4 | 1 | 1 | 2 | 2 | 3 | 3 | 3 | 3 | 3 | 3 | - | 4 | 4 | 2 | 3 |
| 0.5 | (0.56) | 1 | 1 | 1 | 1 | 1 | 1 | 1 | 1 | 1 | 1 | 3 | 1 | 2 | 2 | 2 | - | 2 | 1 | 1 | 3 |

Post-emergence

| appln. rate lbs/A | (kg/ha) | Corn | Large Crabgrass | Pigweed | Foxtail | Velvetleaf | Morningglory | Zinnia |
|---|---|---|---|---|---|---|---|---|
| 2 | (2.71) | 1 | 2 | 5 | 2 | 2 | 2 | 2 |
| 1 | (1.12) | 1 | 2 | 4 | 2 | 1 | 2 | 2 |
| 0.5 | (0.56) | 1 | 2 | 3 | 2 | 1 | 2 | 2 |

Table 9

Plant Injury Ratings for 3-(5-dimethylaminosulfonyl-1,3,4-thiadiazol-2-yl)-4-acetoxy-1-methyl-2-imidazolidinone from Example 9

PLANT SPECIES

Pre-emergence

| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia |
|---|---|---|---|---|---|---|---|---|---|---|---|---|---|---|---|---|---|---|---|---|---|
| 2 | (2.71) | 5 | 4 | 4 | 4 | 5 | 5 | 5 | 5 | 5 | 5 | 5 | 5 | 5 | 5 | 5 | - | 5 | 5 | 5 | 5 |
| 1 | (1.12) | 3 | 5 | 4 | 4 | 5 | 5 | 5 | 5 | 5 | 5 | 5 | 5 | 5 | 5 | 5 | - | 5 | 5 | 5 | 5 |
| 0.5 | (0.56) | 2 | 2 | 1 | 2 | 3 | 3 | 2 | 2 | 3 | 2 | 4 | 5 | 3 | 5 | 5 | 3 | 5 | 4 | 4 | 5 |
| 0.25 | (0.28) | 1 | 1 | 1 | 2 | 1 | 3 | 1 | 1 | 1 | 2 | 3 | 4 | 2 | 3 | 2 | 2 | 3 | 2 | 3 | 3 |

Post-emergence

| appln. rate lbs/A | (kg/ha) | Corn | Large Crabgrass | Pigweed | Foxtail | Velvetleaf | Morningglory | Zinnia |
|---|---|---|---|---|---|---|---|---|
| 2 | (2.71) | 4 | 3 | 5 | 5 | 4 | 5 | 5 |
| 1 | (1.12) | 1 | 3 | 5 | 4 | 3 | 3 | 4 |
| 0.5 | (0.56) | 2 | 2 | 5 | 3 | 2 | 3 | 4 |
| 0.25 | (0.28) | 2 | 2 | 5 | 3 | 2 | 2 | 4 |

Table 8

Plant Injury Ratings for 3-(5-dimethylaminosulfonyl-1,3,4-thiadiazol-2-yl)-1-methyl-2-imidazolinone from Example 8

PLANT SPECIES

| | | Pre-emergence | | | | | | | | | | | | | | | | | | | | Post-emergence | | | | | | |
|---|---|---|---|---|---|---|---|---|---|---|---|---|---|---|---|---|---|---|---|---|---|---|---|---|---|---|---|---|
| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Corn | Large Crabgrass | Pigweed | Foxtail | Velvetleaf | Morningglory | Zinnia |
| 4 | (4.5) | 2 | 3 | 4 | 3 | 5 | 5 | 2 | 5 | 5 | 5 | 5 | 4 | 4 | 5 | 5 | 1 | 5 | 5 | 5 | 5 | - | - | - | - | - | - | - |
| 2 | (2.7) | 1 | 1 | 2 | 1 | 5 | 4 | 1 | 5 | 4 | 4 | 4 | 3 | 2 | 5 | 3 | 1 | 4 | 3 | 4 | 5 | - | - | - | - | - | - | - |
| 1 | (1.12) | 1 | 1 | 1 | 1 | 3 | 3 | 1 | 5 | 3 | 3 | 3 | 3 | 1 | 5 | 2 | 1 | 4 | 3 | 4 | 4 | - | - | - | - | - | - | - |

## Table 7

Plant Injury Ratings for 3-(5-N-methyl-N-methoxyaminosulfonyl-1,3,4-thiadiazol-2-yl)-4-hydroxy-1-methyl-2-imidazolidinone from Example 7

PLANT SPECIES

| | | Pre-emergence | | | | | | | | | | | | | | | | | | | | Post-emergence | | | | | | |
|---|---|---|---|---|---|---|---|---|---|---|---|---|---|---|---|---|---|---|---|---|---|---|---|---|---|---|---|---|
| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Corn | Large Crabgrass | Pigweed | Foxtail | Velvetleaf | Morningglory | Zinnia |
| 4 | (4.5) | 3 | 1 | 1 | 2 | 3 | 4 | 2 | 3 | 2 | 4 | 4 | 4 | 4 | 5 | 5 | 5 | 4 | 4 | 4 | 1 | 1 | 3 | 5 | 3 | 3 | 3 | 4 |
| 2 | (2.7) | 1 | 1 | 2 | 2 | 2 | 4 | 3 | 1 | 5 | 4 | 5 | 4 | 3 | 5 | 5 | 5 | 4 | 4 | 2 | 5 | 3 | 2 | 3 | 2 | 2 | 3 | 2 |
| 1 | (1.12) | 1 | 1 | 1 | 1 | 1 | 4 | 3 | 2 | 1 | 2 | 4 | 5 | 3 | 1 | 3 | 4 | 5 | 3 | 1 | 1 | 1 | 2 | 5 | 2 | 3 | 4 | 4 |
| 0.5 | (0.56) | 1 | 1 | 1 | 1 | 1 | 3 | 1 | 1 | 1 | 1 | - | 3 | 3 | 2 | 4 | 1 | 3 | 2 | 1 | 2 | 2 | 2 | 4 | 3 | 4 | 3 | 4 |
| 0.25 | (0.28) | 1 | 1 | 1 | 1 | 1 | 1 | 1 | 1 | 1 | 1 | - | 3 | 1 | 1 | 2 | 1 | 1 | 1 | 1 | 2 | 3 | 2 | 4 | 4 | 4 | 4 | 5 |

## Table 6

Plant Injury Ratings for 3-[5-N-methyl-N-(2-propynylaminosulfonyl-1,3,4-thiadiazol-2-yl]-4-hydroxy-1-methyl-2-imidazolidinone from Example 6

PLANT SPECIES

| | | Pre-emergence | | | | | | | | | | | | | | | | | | Post-emergence | | | | | | |
|---|---|---|---|---|---|---|---|---|---|---|---|---|---|---|---|---|---|---|---|---|---|---|---|---|---|---|
| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Corn | Large Crabgrass | Pigweed | Foxtail | Velvetleaf | Morningglory | Zinnia |
| 2 | (2.7) | 1 | 1 | 1 | 1 | 2 | 5 | 4 | 1 | 1 | 3 | 5 | 4 | 4 | 4 | 5 | 4 | 5 | 4 | 3 | 4 | 1 | 4 | 5 | 4 | 5 | 3 | 4 |
| 1 | (1.12) | 1 | 1 | 1 | 1 | 5 | 4 | 3 | 1 | 3 | 4 | 5 | 5 | 5 | 4 | 5 | 5 | 5 | 5 | 3 | 4 | 1 | 3 | 4 | 2 | 3 | 3 | 2 |
| 0.5 | (0.56) | 1 | 1 | 1 | 1 | 1 | 2 | 1 | 1 | 1 | 4 | - | 3 | 2 | 1 | 2 | 1 | 1 | 2 | 1 | 1 | | | | | | | |
| 0.25 | (0.28) | 1 | 1 | 1 | 1 | 1 | 1 | 1 | 1 | 1 | 1 | - | 4 | 3 | 1 | 2 | 1 | 1 | 1 | 1 | 1 | | | | | | | |

Table 5

Plant Injury Ratings for 3-[5-N-methyl-N-(n-butyl)aminosulfonyl-1,3,4-thiadiazol-2-yl]-4-hydroxy-1-methyl-2-imidazolidinone from Example 5

PLANT SPECIES

| | | Pre-emergence | | | | | | | | | | | | | | | | | | | | Post-emergence | | | | | | | |
|---|---|---|---|---|---|---|---|---|---|---|---|---|---|---|---|---|---|---|---|---|---|---|---|---|---|---|---|---|---|
| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Tomato | Corn | Large Crabgrass | Pigweed | Foxtail | Velvetleaf | Morningglory | Zinnia |
| 15 | (16.8) | - | - | - | - | - | - | - | - | 4 | - | - | 2 | - | 3 | - | - | - | - | - | - | 5 | - | 5 | 5 | - | - | - | - |
| 8 | (8.97) | 5 | - | - | - | - | - | - | - | - | - | - | 5 | - | 5 | 5 | - | 5 | - | 5 | 5 | - | 2 | 3 | 4 | 3 | 4 | 3 | 4 |
| 4 | (4.5) | 3 | 2 | 1 | 2 | 2 | 4 | 2 | 3 | 4 | 5 | 5 | 5 | 5 | 5 | 5 | 5 | 5 | 5 | 5 | 5 | - | 3 | 3 | 5 | 5 | 4 | 3 | 4 |
| 2 | (2.7) | 1 | 2 | 1 | 2 | 1 | 2 | 2 | 3 | 3 | 4 | 5 | 5 | 3 | 4 | 5 | 4 | 4 | - | 4 | 3 | - | 3 | 4 | 4 | 3 | 3 | 4 | 5 |
| 1 | (1.12) | 1 | 1 | 2 | 2 | 1 | 3 | 1 | 2 | 3 | 4 | 5 | 5 | 3 | 4 | 4 | 4 | 2 | 5 | 3 | 4 | - | 3 | 2 | 4 | 2 | 3 | 3 | 3 |

Table 4

Plant Injury Ratings for 3-[5-N-methyl-N-(n-propyl)aminosulfonyl-1,3,4-thiadiazol-2-yl]-4-hydroxy-1-methyl-2-imidazolidinone from Example 4

| appln. rate lbs/A | (kg/ha) | PLANT SPECIES Pre-emergence | | | | | | | | | | | | | | | | | | | | Post-emergence | | | | | | |
|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Corn | Large Crabgrass | Pigweed | Foxtail | Velvetleaf | Morningglory | Zinnia |
| 4 | (4.5) | 4 | 5 | 4 | 5 | 5 | 5 | 4 | 5 | 5 | 5 | 5 | 5 | 5 | 5 | 5 | 5 | 5 | 5 | 4 | 5 | 4 | 4 | 5 | 4 | 3 | 4 | 4 |
| 2 | (2.7) | 3 | 4 | 4 | 4 | 5 | 5 | 5 | 5 | 5 | 5 | 5 | 5 | 5 | 5 | 5 | 5 | 5 | 5 | 5 | 5 | 2 | 4 | 5 | 5 | 4 | 4 | 5 |
| 1 | (1.12) | 3 | 1 | 1 | 3 | 4 | 4 | 3 | 4 | 5 | 5 | 5 | 5 | 5 | 5 | 4 | 5 | 5 | 4 | 4 | 5 | 2 | 4 | 5 | 3 | 3 | 4 | 4 |
| 0.5 | (0.56) | 1 | 1 | 2 | 4 | 4 | 5 | 2 | 4 | 4 | 4 | 5 | 4 | 4 | 4 | 5 | 3 | 5 | 4 | 4 | 5 | 1 | 3 | 5 | 4 | 3 | 2 | 3 |
| 0.25 | (0.28) | 1 | 1 | 1 | 2 | 2 | 3 | 1 | 1 | 1 | 3 | 5 | 4 | 3 | 3 | 3 | 2 | 5 | 4 | 4 | 5 | 1 | 3 | 5 | 4 | 3 | 2 | 3 |
| 0.125 | (0.14) | 1 | 1 | 1 | 1 | 1 | 1 | 1 | 2 | 1 | 1 | 1 | 2 | 1 | 1 | 1 | 1 | 1 | 1 | 1 | 1 | 2 | 3 | 4 | 4 | 2 | 3 | 2 |
| 0.0625 | (0.07) | 1 | 1 | 1 | 1 | 1 | 1 | 1 | 1 | 1 | 1 | 1 | 1 | 1 | 1 | 1 | 1 | 1 | 1 | 1 | 1 | 2 | 3 | 4 | 3 | 2 | 2 | 3 |

## Table 3

Plant Injury Ratings for 3-(5-N-ethyl-N-methylaminosulfonyl-1,3,4-thiadiazol-2-yl)-4-hydroxy-1-methyl-2-imidazolidinone from Example 3

PLANT SPECIES

Pre-emergence

| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia |
|---|---|---|---|---|---|---|---|---|---|---|---|---|---|---|---|---|---|---|---|---|---|
| 4 | (4.5) | 5 | 5 | 5 | 5 | 5 | 5 | 5 | 5 | 5 | 5 | 5 | 5 | 5 | 5 | 5 | 5 | 5 | 3 | 5 | 5 |
| 2 | (2.7) | 4 | 5 | 4 | 5 | 5 | 5 | 5 | 5 | 5 | 5 | 5 | 5 | 5 | 5 | 5 | 5 | 5 | 3 | 5 | 5 |
| 1 | (1.12) | 3 | 1 | 2 | 4 | 5 | 5 | 5 | 5 | 5 | 5 | 5 | 5 | 5 | 5 | 5 | 5 | 5 | 5 | 5 | 5 |
| 0.5 | (0.56) | 1 | 1 | 2 | 4 | 5 | 5 | 3 | 5 | 5 | 5 | 5 | 4 | 5 | 5 | 5 | 4 | 4 | 5 | 5 | 5 |
| 0.25 | (0.28) | 1 | 1 | 1 | 3 | 4 | 3 | 2 | 3 | 2 | 4 | 4 | 4 | 4 | 4 | 5 | 2 | 4 | 4 | 3 | 4 |

Post-emergence

| appln. rate lbs/A | (kg/ha) | Corn | Large Crabgrass | Pigweed | Foxtail | Velvetleaf | Morningglory | Zinnia |
|---|---|---|---|---|---|---|---|---|
| 4 | (4.5) | 4 | 5 | 5 | 5 | 5 | 5 | 5 |
| 2 | (2.7) | 4 | 5 | 5 | 5 | 5 | 5 | 5 |
| 1 | (1.12) | 3 | 4 | 5 | 5 | 4 | 4 | 5 |
| 0.5 | (0.56) | 2 | 3 | 5 | 4 | 3 | 3 | 4 |
| 0.25 | (0.28) | 1 | 3 | 5 | 4 | 3 | 3 | 3 |

## Table 2

Plant Injury Ratings for 3-(5-diethylaminosulfonyl-1,3,4-thiadiazol-2-yl)-4-hydroxy-1-methyl-2-imidazolidinone from Example 2

PLANT SPECIES

| | | Pre-emergence | | | | | | | | | | | | | | | | | | | | Post-emergence | | | | | | |
|---|---|---|---|---|---|---|---|---|---|---|---|---|---|---|---|---|---|---|---|---|---|---|---|---|---|---|---|---|
| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Corn | Large Crabgrass | Pigweed | Foxtail | Velvetleaf | Morningglory | Zinnia |
| 4 | (4.5) | 4 | 2 | 2 | 3 | 2 | 5 | 3 | 5 | 4 | 5 | 5 | 5 | 4 | 4 | 4 | 4 | 5 | 4 | 5 | 5 | 4 | 3 | 4 | 4 | 4 | 4 | 4 |
| 2 | (2.7) | 2 | 2 | 1 | 1 | 2 | 4 | 2 | 4 | 4 | 3 | 5 | 4 | 4 | 5 | 5 | 4 | 5 | 4 | 4 | 5 | 5 | 4 | 5 | 4 | 5 | 4 | 4 |
| 1 | (1.12) | 1 | 1 | 1 | 2 | 1 | 5 | 2 | 2 | 2 | 3 | 5 | 5 | 3 | 4 | 4 | 5 | 5 | - | 3 | 2 | 4 | 3 | 3 | 3 | 4 | 4 | 4 |
| 0.5 | (0.56) | 1 | 1 | 1 | 2 | 1 | 3 | 3 | 2 | 3 | 2 | - | 5 | 4 | 5 | 5 | 2 | 4 | 4 | 3 | 4 | 1 | 3 | 3 | 5 | 3 | 3 | 5 |

## Table 1

Plant Injury Ratings for 3-(5-dimethylaminosulfonyl-1,3,4-thiadiazol-2-yl)-4-hydroxy-1-methyl-2-imidazolidinone from Example 1

PLANT SPECIES

| | | Pre-emergence | | | | | | | | | | | | | | | | | | | | Post-emergence | | | | | | |
|---|---|---|---|---|---|---|---|---|---|---|---|---|---|---|---|---|---|---|---|---|---|---|---|---|---|---|---|---|
| appln. rate lbs/A | (kg/ha) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia | Corn | Large Crabgrass | Pigweed | Foxtail | Velvetleaf | Morningglory | Zinnia |
| 4 | (4.5) | 5 | 5 | 4 | 5 | 5 | 5 | 5 | 5 | 5 | 5 | 5 | 5 | 5 | 5 | 5 | 5 | 5 | 5 | 5 | 5 | 4 | 5 | 5 | 5 | 5 | 4 | 5 |
| 2 | (2.7) | 4 | 5 | 4 | 5 | 5 | 5 | 5 | 5 | 5 | 5 | 5 | 5 | 5 | 5 | 5 | 5 | 5 | 5 | 5 | 5 | 3 | 5 | 5 | 4 | 5 | 4 | 5 |
| 1 | (1.12) | 3 | 3 | 2 | 3 | 5 | 5 | 5 | 5 | 5 | 5 | 5 | 5 | 5 | 5 | 5 | 5 | 5 | 5 | 5 | 5 | 1 | 2 | 4 | 3 | 2 | 2 | 2 |
| 0.5 | (0.56) | 2 | 1 | 1 | 3 | 3 | 4 | 4 | 5 | 5 | 3 | 5 | 5 | 4 | 5 | 5 | 4 | 5 | 5 | 4 | 5 | | | | | | | |
| 0.25 | (0.28) | 1 | 1 | 1 | 1 | 1 | 3 | 2 | 4 | 3 | 3 | 1 | 4 | 2 | 3 | 3 | 2 | 2 | 4 | 1 | 4 | | | | | | | |
| 0.125 | (0.14) | 1 | 1 | 1 | 1 | 1 | 1 | 1 | 1 | 1 | 2 | 2 | 3 | 2 | 3 | 3 | 2 | 1 | 1 | 2 | 2 | | | | | | | |
| 0.0625 | (0.07) | 1 | 1 | 1 | 1 | 1 | 1 | 1 | 1 | 1 | 1 | 1 | 1 | 1 | 2 | 1 | 1 | 1 | 1 | 1 | 1 | | | | | | | |

injury; "3" moderate injury; "4" severe injury; and "5" death to all treated plants. A dash "-" in any column means that no evaluation was made at that level on the particular plant species. Evaluations were made from 11 to 14 days after post-emergence application, and from 18 to 21 days following pre-emergence application.

Tables 1-15 which follow presents the herbicidal activity of typical compounds provided by this invention.

The data presented in Table 1 demonstrate that 3-(5-dimethylaminosulfonyl-1,3,4-thiadiazol-2-yl)-4-hydroxy-1-methyl-2-imidazolidinone is effective both as a pre-emergence and a post-emergence terrestrial herbicide at rates as low as about 1 pound per acre (1.12 kg/ha) against weeds such as velvetleaf, zinnia, wildoat, foxtail, pigweed and lambsquarter, while having no or only slight adverse affect on desirable plants such as corn, cotton and soybean. The compound is active as a pre-emergence herbicide at rates as low as 0.125 pound per acre (0.14 kg/ha).

The data presented in Table 2 demonstrate that the compound tested is an excellent pre- and post-emergence herbicide at rates as low as 0.5 pound per acre (0.56 kg/ha) against commonly occurring terrestrial weeds such as lambsquarter and velvetleaf, while displaying little or no toxic affects to desirable plants such as corn, wheat, rice, alfalfa and the like.

Similarly, the data of Table 4 show that a thiadiazolylimidazolidinone of this invention is toxic both pre- and post-emergence at rates as low as 0.25 pound per acre (0.28 kg/ha) against weeds such as lambsquarter, crabgrass, and pigweed, while having little or no adverse affect on corn, cotton, soybean, rice, or the like.

The data thus far generated indicate that compounds embraced by this invention are useful as selective terrestrial herbicides against common weed varities which are found in desirable crops such as

corn, wheat, soybean, and the like. The compounds can also be used in a chemical fallow program, for example in a fallow wheatland weed control program.

Thus, a further important aspect of the invention is that it provides a method for controlling the growth of vegetation which comprises applying to the locus where vegetative control is desired a herbicidally effective amount of a thiadiazolylimidazolidinone derivative of formula (I) as defined above.

The practice of the foregoing method can be carried out by applying the herbicidal compounds by any of several art-recognized methods. The compounds can be surface applied to soil prior to planting of crops or emergence of seedlings. If desired, the surface applied herbicide can be incorporated into the soil by conventional means such as a double disc or harrow. The compounds can alternatively be applied early post-emergence, for instance within about 2 weeks following seedling emergence.

The amount of herbicidal thiadiazolylimidazolidinone derivatives to be employed in the method of this invention is an amount which is effective in controlling the growth of unwanted vegetation. Such herbicidal amount will depend upon a number of factors, including the method of application, formulation, soil texture, soil moisture content, the expected population of unwanted vegetation, degree of incorporation, the extent of growth control desired, and related factors. The rate of application normally will be from about 0.01 to about 10.0 pounds per acre, and preferably from about 0.25 to about 5.0 pounds per acre. These ranges

are equivalent, respectively, to from about 0.011 to about 11.2 kilograms per hectare, and from about 0.28 to about 5.6 kilograms per hectare.

The thiadiazolylimidazolidinone and thiadiazolylimidazolinone herbicides to be employed according to the terrestrial method of this invention can be formulated for convenient application as aqueous sprays, drenches, solid powders, dusts and the like. Herbicidal formulations containing a thiadiazolylimidazolidinone derivative as defined herein are provided in a further embodiment of this invention. Such formulations will contain from about 1.0 to about 90 percent by weight of active thiadiazolylimidazolidinone derivative, and can be prepared by combining a thiadiazolylimidazolidinone derivative with any number of common agronomically acceptable carriers, diluents, adjuvants and the like. The formulations can contain liquid carriers and adjuvants such as organic solvents, including benzene, xylene, toluene, a halo-toluene such as <u>ortho</u>-chlorotoluene, <u>n</u>-hexane, <u>n</u>-pentane, kerosene, and similar organic solvents. The formulations can additionally contain art-known emulsifiers, stabilizers, dispersants, suspending agents, spreaders, penetrants, wetting agents and the like.

Typical carriers utilized in the dry formulations of the invention include clay, talc, diatomaceous earth, silica, pyrophyllite and the like. Herbicidal dusts are prepared by grinding and blending such solid carriers with a compound of the invention such as 3-(5-dimethylaminosulfonyl-1,3,4-thiadiazol-2-yl)-4-hydroxy-5-ethyl-1-methyl-2-imidazolidinone. Granular formulations provided herein are prepared by impregnating a thiadiazolylimidazolidinone derivative onto or into a granulated carrier such as a vermiculite. Such granules typically have a particle size of about 0.2 to about 2.0 mm.

Preferred formulations of the inventions are in the form of wettable powders. Such formulations employ any number of commercially available wetting agents, surfactants and emulsifying agents such as ethoxylated nonyl phenols, ethoxylated octyl phenols, polyoxyethylene stearyl ethers, blends of such agents, and the like. Such formulations can optionally contain adjuvants such as thickening agents, for instance heteropolysaccharide gums and the like such as xanthan gum, as well as antibacterial agents such as aqueous formaldehyde.

Thus, yet another aspect of the invention is that it provides a herbicidal formulation comprising as an active ingredient from 1.0 to 90.0 wt. % of a compound of formula (I) as defined above associated with at least one carrier or diluent therefor.

The following detailed examples of formulations illustrate preferred aspects of the invention.

Example 17

Granule

| Ingredient | Percent by Weight |
|---|---|
| 3-(5-diethylaminosulfonyl-1,3,4-thiadiazol-2-yl)-4-hydroxy-1-ethyl-2-imidazolidinone | 5.0 |
| clay chips | 95.0 |
| | 100.0 percent |

The thiadiazolylimidazolidinone herbicide is substantially dissolved in acetone or similar solvent, and the organic solution is sprayed onto the clay chips. The mixture is thoroughly blended and dried.

Example 18

Dust

| Ingredient | Percent by Weight |
|---|---|
| 3-(5-dimethylaminosulfonyl-1,3,4-thiadiazol-2-yl)-4-acetoxy-5-n-butyl-1-(1,1-dimethylpropyl)-2-imidazolidinone | 40.0 |
| powdered talc | 60.0 |
| | 100.0 percent |

The ingredients are mixed and blended to uniformity and are ground and air-milled to a homogenous, free-flowing dust. The dust may be applied directly to the locus of weed infestation.

Example 19

Wettable Powder

| Ingredient | Percent by Weight |
|---|---|
| 3-(5-dimethylaminosulfonyl-1,3,4-thiadiazol-2-yl)-4-hydroxy-1-methyl-2-imidazolidinone | 50.0 |
| Fuller's earth | 47.0 |
| methyl cellulose | 0.5 |
| sodium lauryl sulfate | 2.5 |
| | 100.0 percent |

The above ingredients are blended to uniformity and dispersed in water to the desired concentration of active ingredient.

The herbicidal thiadiazolylimidazolidinone derivatives of this invention can be employed in combination with other herbicides for the effective control of an even broader spectrum of weeds. Well known herbicides which may be utilized together with the herbicides of this invention include diuron, atrazine, trifluralin, metribuzin, simazine, propazine, paraquat, and the like. Combinations of the compounds of this invention together with herbicides which are especially effective against grasses are particularly preferred. Typical of such combinations is trifluralin and a compound such as 3-(5-dimethylaminosulfonyl-1,3,4-thiadiazol-2-yl)-1-ethyl-2-imidazolinone. It is contemplated that such combinations will contain the respective herbicides in approximately equal amounts, and that upon application each respective herbicide will be present at about 0.1 to about 5.0 pounds per acre (0.112 to about 5.6 kg/ha).

The thiadiazolylimidazolidinones of this invention also are useful in the control of unwanted vegetation in a chemical fallow program, for instance in the control of lambsquarter, morningglory, velvetleaf, and similar weeds in fallow wheatland. Combinations with herbicides such as atrazine and trifluralin are particularly useful in a fallow program.

The compounds defined herein also are useful in regulating the growth pattern of plant life without causing total elimination. In order to achieve such

plant growth regulation, the compounds are employed in amounts which are effective to cause growth regulation but are not herbicidal. The compounds are applied directly to the plants or to the locus where the plants to be regulated are growing. The compounds are preferably formulated for post-emergence over the top spray application. Typical manifestations of plant growth regulation employing compounds of this invention include stunting, control of flowering, control of fruiting, delay in maturation, reduction in stem growth, improvements in yield, and related regulatory responses.

The plant growth regulating properties of the compounds defined herein have been determined by the following test procedure. Test compounds were formulated as a foliar spray by dissolving 50 mg in 25 ml of Toximul R and Toximul S (50:50). An aliquot of the solution was further diluted to a concentration of test compound of 1000 parts per million (ppm). The formulated test compound was sprayed onto the leaves of squash and bean plants, and the plants were then allowed to dry for two hours. All leaves were then removed from the treated plants, and the plants were placed in a greenhouse. Evaluations for terminal growth and morphological effects were made after five days. Growth regulator activity is rated on a scale of +3 to -3. A "+" indicates growth promotion, while a "-" indicates growth inhibition. The numerical ratings are assigned the meanings:

0 = no effect
1 = slight response
2 = moderate response
3 = distinct response.

Phytotoxicity is indicated according to the following scale:

B = burn
C = chlorosis
X = death

The results of plant growth regulator tests for several representative compounds of the invention are presented in Tables 16, 17, and 18 which follow:

Table 16

Plant Growth Regulator Activity

| Compound of Example No. | Concentration ppm | Squash Growth Rating | Squash Phyto-toxicity | Bean Growth Rating | Bean Phyto-Toxicity |
|---|---|---|---|---|---|
| 4 | 1000 | -1 | 0 | -2 | 0 |
| 5 | 1000 | -2 | 0 | -3 | 3C |
| 7 | 1000 | 0 | 0 | -3 | 3C |
| 13 | 1000 | -1 | 0 | -2 | 0 |

Table 17

Plant Growth Regulator Activity

| Compound of Example No. | Concentration ppm | Snap bean Shoot Growth | Snap bean Root Growth |
|---|---|---|---|
| 5 | 100 | -1 | -2 |
| 7 | 100 | -3 | -3 |
| 11 | 100 | -1 | 0 |

Table 18

Plant Growth Regulator Activity

| Compound of Example No. | Concen-tration | Mode of Application | Plant Variety | Epinasty | Abscission | Height | Branching | Injury |
|---|---|---|---|---|---|---|---|---|
| 4 | 2000 | foliar | cotton | 0 | - | -3 | 0 | 3X |
| | | | soybean | 2 | - | -3 | 0 | 3X |
| | | | cucumber | 0 | - | -3 | - | 3X |
| | | | barley | - | - | -3 | - | 3X |
| | | | ryegrass | - | - | -3 | - | 3X |
| 4 | 5 | soil | cotton | 0 | 3 | -3 | 0 | 3X |
| | | | soybean | 0 | - | -3 | 0 | 3X |
| | | | cucumber | 0 | - | -3 | - | 3X |
| | | | barley | - | - | -3 | - | 3X |
| | | | ryegrass | - | - | -3 | - | 3X |

Table 18 (continued)

Plant Growth Regulator Activity

| Compound of Example No. | Concentration | Mode of Application | Plant Variety | Epinasty | Alscission | Height | Branching | Injury |
|---|---|---|---|---|---|---|---|---|
| 5 | 2000 | foliar | cotton | 0 | 0 | -2 | 0 | 2B |
| | | | soybean | 0 | - | -3 | 0 | 2X |
| | | | cucumber | 0 | - | -3 | 0 | 3X |
| | | | barley | - | - | -3 | - | 3X |
| | | | ryegrass | - | - | -2 | - | 2B |
| 5 | 50 | soil | cotton | 0 | 0 | -3 | 0 | 3X |
| | | | soybean | 0 | - | -3 | 0 | 3X |
| | | | cucumber | 0 | - | -3 | 0 | 3X |
| | | | barley | - | - | -3 | - | 3X |
| | | | ryegrass | - | - | -2 | - | 2B |

As the data in Tables 16, 17, and 18 demonstrate, the compounds of the invention are useful in controlling the growth of plants when applied at non-herbicidal rates. While the particular rate will vary depending upon the growth regulator response desired and the plant species treated, a typical plant growth regulator amount will be from about 0.01 to about 5.0 pounds per acre.

The thiadiazolylimidazolidinones of this invention have additionally demonstrated good aquatic herbicidal and plant growth regulator activity, and thus are useful in controlling the growth of aquatic vegetation such as hydrilla, coontail, chara, naiad, duckweed and the like. The compounds are also effective as aquatic algicides and can be employed to control algae such as Chlorella vulgaris, Anacystis nidulans and the like.

## CLAIMS

1. A thiadiazolylimidazolidinone derivative of the formula (I)

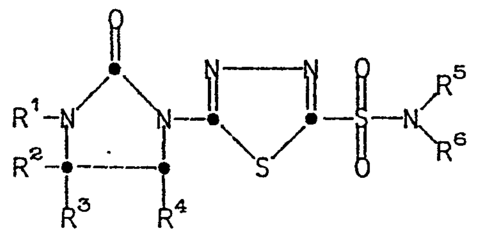

(I).

wherein:

$R^1$ is hydrogen, $C_1$-$C_6$ alkyl, or phenyl;

$R^2$ is hydrogen, or taken together with $R^4$ completes a double bond;

$R^3$ is hydrogen or $C_1$-$C_6$ alkyl;

$R^4$ is hydroxy, $C_1$-$C_6$ alkanoyloxy, $C_1$-$C_6$ alkoxy, di($C_1$-$C_6$ alkyl)amino, $O\overset{O}{\overset{\|}{C}}NR^1R^b$, where $R^b$ is hydrogen or $C_1$-$C_6$ alkyl and $R^1$ is as defined above; or taken together with $R^2$, $R^4$ completes a double bond;

$R^5$ is hydrogen or $C_1$-$C_6$ alkyl; and

$R^6$ is hydrogen, $C_1$-$C_6$ alkyl, $C_2$-$C_6$ alkenyl, $C_2$-$C_6$ alkynyl, $C_1$-$C_6$ alkoxy, or taken together with the nitrogen to which they are attached, $R^5$ and $R^6$ are pyrrolidino, piperidino or morpholino.

2. The compound of Claim 1 wherein $R^2$ and $R^4$ are taken together to complete a double bond.

3. A thiadiazolylimidazolidinone derivative of the formula (I) as claimed in claim 1 selected from

3-(5-dimethylaminosulfonyl-1,3,4-thiadiazol-2-yl)-4-hydroxy-1-methyl-2-imidazolidinone;

3-(5-diethylaminosulfonyl-1,3,4-thiadiazol-2-yl)-4-hydroxy-1-methyl-2-imidazolidinone,

3-(5-N-methyl-N-ethylaminosulfonyl-1,3,4-thiadiazol-2-yl)-4-hydroxy-1-methyl-2-imidazolidinone,

3-(5-N-methyl)-N-propylaminosulfonyl-1,3,4-thiadiazol-2-yl-4-hydroxy-1-methyl-2-imidazolidinone,

3-[5-N-methyl-N-(n-butyl)aminosulfonyl-1,3,4-thiadiazol-2-yl]-4-hydroxy-1-methyl-2-imidazolidinone,

3-[5-N-methyl-N-(2-propynyl)aminosulfonyl-1,3,4-thiadiazol-2-yl]-4-hydroxy-1-methyl-2-imidazolidinone, .

3-(5-N-methyl-N-methoxyaminosulfonyl-1,3,4-thiadiazol-2-yl)-4-hydroxy-1-methyl-2-imidazolidinone,

3-(5-dimethylaminosulfonyl-1,3,4-thiadiazol-2-yl)-1-methyl-2-imidazolinone,

3-(5-dimethylaminosulfonyl-1,3,4-thiadiazol-2-yl)-4-acetoxy-1-methyl-2-imidazolidinone,

3-(5-dimethylaminosulfonyl-1,3,4-thiadiazol-2-yl)-4-methoxy-1-methyl-2-imidazolidinone,

3-(5-dimethylaminosulfonyl-1,3,4-thiadiazol-2-yl)-4-hydroxy-2-imidazolidinone,

3-(5-dimethylaminosulfonyl-1,3,4-thiadiazol-2-yl)-4-hydroxy-1-phenyl-2-imidazolidinone,

3-(5-dimethylaminosulfonyl-1,3,4-thiadiazol-2-yl)-4-methylaminocarbonyloxy-1-methyl-2-imidazolidinone,

3-(5-dimethylaminosulfonyl-1,3,4-thiadiazol-2-yl)-4-phenylaminocarbonyl-1-methyl-2-imidazolidinone,

3-(5-dimethylaminosulfonyl-1,3,4-thiadiazol-2-yl)-4-dimethylamino-1-methyl-2-imidazolidinone,

3-(5-dimethylaminosulfonyl-1,3,4-thiadiazol-2-yl)-4-hydroxy-1-methyl-2-imidazolidinone.

4. A thiadiazolylimidazolidinone derivative of formula (I) in accordance with Claim 1 or 3 wherein $R^4$ is hydroxy.

5. A thiadiazolylimidazolidinone derivative of formula (I) as claimed in any one of Claims 1-4 for use as an herbicide.

6. A herbicidal formulation comprising, as an active ingredient from 1.0 to 90.0 wt. % of a compound of formula (I) as claimed in any one of Claims 1-4 associated with at least one carrier or diluent therefor.

7. A herbicidal formulation as claimed in Claim 5 which is a wettable powder..

8. A process for preparing a thiadiazolylimidazolidinone derivative of formula (I) as described in Claim 1, which comprises the steps of

(a) heating a (dialkoxy)ethyl thiadiazolylurea of the formula (II):

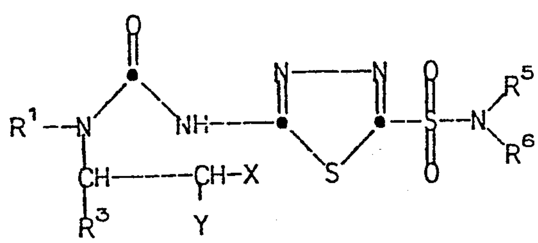

(II)

wherein:

$R^1$ is hydrogen, $C_1$-$C_6$ alkyl, or phenyl,

$R^3$ is hydrogen or $C_1$-$C_6$ alkyl,

$R^5$ is hydrogen or $C_1$-$C_6$ alkyl, and

$R^6$ is hydrogen, $C_1$-$C_6$ alkyl, $C_2$-$C_6$ alkenyl, $C_2$-$C_6$ alkynyl, $C_1$-$C_6$ alkoxy, or

$R^5$ and $R^6$ taken together with the nitrogen to which they are attacked form pyrrolidino, piperidino, or morpholino, and

X and Y independently are $C_1$-$C_6$ alkoxy; in the presence of an inorganic or an organic acid; or

(b) dehydrating a thiadiazolylimidazolidinone derivative of formula (I) wherein $R^4$ is hydroxy to provide a thiadiazolylimidazolidinone derivative of formula (I) wherein $R^2$ and $R^4$ combine to complete a double bond; or

(c) reacting a thiadiazolylimidazolidinone derivative of formula (I) wherein $R^4$ is hydroxy with an acylating agent derived from a $C_1$-$C_6$ carboxylic acid to provide a thiadiazolylimidazolidinone derivative of formula (I) wherein $R^4$ is $C_1$-$C_6$ alkanoyloxy; or

(d) reacting a thiadiazolylimidazolidinone derivative of formula (I) wherein $R^4$ is hydroxy with an isocyanate of the formula $R^1NCO$ or a carbamoyl halide of the formula $R^1R^6NCOCl$, wherein $R^1$ is as defined above and $R^6$ is hydrogen or $C_1$-$C_6$ alkyl, to provide a thiadiazolylimidazolidinone derivative of formula (I) wherein $R^4$ is $O-\overset{O}{\overset{\|}{C}}NR^1R^6$; or

(e) reacting a thiadiazolylimidazolidinone derivative of formula (I) wherein $R^4$ is hydroxy with an amine of the formula

di($C_1$-$C_6$ alkyl)-NH

to provide a thiadiazolylimidazolidinone derivative of formula (I) wherein $R^4$ is di($C_1$-$C_6$ alkyl)amino; or

(f) reacting a thiadiazolylimidazolidinone derivative of formula (I) wherein $R^4$ is hydroxy with a $C_1$-$C_6$ alkyl alcohol in the presence of a mineral acid to provide a thiadiazolylimidazolidinone derivative of formula (I) wherein $R^4$ is $C_1$-$C_6$ alkoxy.

9. A method for controlling the growth of vegetation which comprises applying to the locus where vegetative control is desired a herbicidally effective amount of a thiadiazolylimidazolidinone derivative of formula (I) as claimed in any one of Claims 1-4.

10. A (dialkoxy)ethyl thiadiazolylurea of the formula (II):

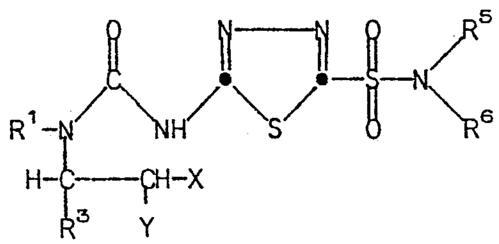

(II)

wherein:

$R^1$ is hydrogen or $C_1$-$C_6$ alkyl;

$R^3$ is hydrogen or $C_1$-$C_6$ alkyl;

$R^5$ is hydrogen or $C_1$-$C_6$ alkyl, and

$R^6$ is hydrogen, $C_1$-$C_6$ alkyl, $C_2$-$C_6$ alkenyl, $C_2$-$C_6$ alkynyl, $C_1$-$C_6$ alkoxy, or

$R^5$ and $R^6$ taken together with the nitrogen atom to which they are attached form pyrrolidino, piperidino, or morpholino, and

X and Y independently are $C_1$-$C_6$ alkoxy.

4i

0093589

European Patent Office

# EUROPEAN SEARCH REPORT

Application number

EP 83 30 2428

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | DE-A-2 247 266 (CIBA GEIGY)<br>* Claims *<br>--- | 1,5-9 | C 07 D 417/04<br>C 07 D 285/12<br>A 01 N 43/82 |
| Y | US-A-4 036 848 (J. KRENZER)<br>* Whole document *<br>--- | 1,5-9 | |
| Y | US-A-4 033 753 (J. KRENZER)<br>* Whole document *<br>--- | 1,5-9 | |
| Y | US-A-3 990 882 (J. KRENZER)<br>* Whole document *<br>--- | 1,2,5-9 | |
| Y | US-A-3 944 409 (J. KRENZER)<br>* Whole document *<br>--- | 1,2,5-9 | TECHNICAL FIELDS SEARCHED (Int. Cl. 3) |
| Y | US-A-3 925 402 (J. KRENZER)<br>* Whole document *<br>--- | 1,5-9 | C 07 D 417/00<br>C 07 D 285/00<br>A 01 N 43/00 |
| Y | US-A-3 856 503 (T. CELABO)<br>* Whole document *<br>----- | 10 | |

The present search report has been drawn up for all claims

| Place of search | Date of completion of the search | Examiner |
|---|---|---|
| THE HAGUE | 15-08-1983 | CREMERS K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document